# FASCICULE DE BREVET EUROPEEN

(11) **EP 1 958 610 B1**
(45) Date de publication et mention de la délivrance du brevet: **10.08.2016**
(21) Numéro de dépôt: 08300087.7
(22) Date de dépôt: 12.02.2008
(51) Int. Cl.: A61K 8/22, A61K 8/81, A61Q 5/04

(54) **Procédé de déformation permanente des fibres kératiniques comprenant une étape d'application d'une composition oxydante contenant un polymère épaississant cationique**
Verfahren zur dauerhaften Verformung von Keratinfasern, das eine Anwendungsphase mit einer oxidierenden Zusammensetzung umfasst, die ein verdickendes kationisches Polymer enthält
Method of permanently deforming keratinous fibres including an application step involving an oxidising composition containing a cationic thickening polymer

(30) Priorité: 13.02.2007 FR 0753237
(43) Date de publication de la demande: 20.08.2008
(73) Titulaire: L'Oréal, 75008 Paris (FR)
(72) Inventeur: Sow, Fatoumata, 92110, Clichy (FR); Sabbagh, Anne, 92500, Rueil Malmaison (FR)
(74) Mandataire: Caillet, Isabelle

(56) Documents cités:
- WO-A-2004/024779
- NN: "Carbopol(TM) Aqua CC Polymer" LUBRIZOL - NOVEON CONSUMER SPECIALTIES, [Online] 7 mars 2006 (2006-03-07), pages 1-2, XP002452401 Extrait de l'Internet: URL:http://www.personalcare.noveon.com/Pro ductSheets/CP-36_Carbopol_Aqua_CC_Product_ Summary.pdf> [extrait le 2007-09-25]

## Description

La présente invention se rapporte à un procédé de déformation permanente des fibres kératiniques, en particulier des cheveux, comprenant une étape de réduction et une étape d'oxydation, l'étape d'oxydation étant effectuée au moyen d'une composition oxydante gélifiée comprenant un polymère cationique spécifique.

La technique la plus usuelle pour obtenir une déformation permanente des cheveux consiste, dans un premier temps, à réaliser l'ouverture des liaisons disulfures -S-S- de la kératine (cystine) à l'aide d'une composition contenant un agent réducteur adapté (étape de réduction) puis, après avoir rincé la chevelure ainsi traitée, à reconstituer dans un second temps lesdites liaisons disulfures en appliquant, sur les cheveux préalablement mis sous tension (bigoudis et autres) ou mis en forme ou lissés par d'autres moyens, une composition oxydante (étape d'oxydation, dite aussi de fixation) de façon à donner finalement aux cheveux la forme recherchée. Cette technique permet ainsi de réaliser indifféremment soit l'ondulation des cheveux (procédé de permanente), soit leur défrisage ou leur décrêpage (procédé de lissage). La nouvelle forme imposée aux cheveux par un traitement chimique tel que ci-dessus est éminemment durable dans le temps et résiste notamment à l'action des lavages à l'eau ou par shampooings, et ceci par opposition aux simples techniques classiques de déformation temporaire, telles que de mise en pli.

Les compositions réductrices utilisables pour la mise en oeuvre de la première étape d'une opération de permanente contiennent généralement, à titre d'agents réducteurs, des sulfites, des bisulfites, des alkyl-phosphines ou de préférence des thiols. Parmi ces derniers, ceux couramment utilisés sont la cystéïne et ses divers dérivés, la cystéamine et ses dérivés, l'acide thiolactique ou l'acide thioglycolique, leurs sels ainsi que leurs esters, notamment le thioglycolate de glycérol.

Les compositions oxydantes nécessaires à la mise en oeuvre de l'étape de fixation sont le plus souvent des compositions à base d'eau oxygénée ou de bromate de métaux alcalins.

Dans les procédés de déformation permanente des cheveux, en particulier dans les procédés de permanente, les compositions oxydantes sont le plus souvent sous forme liquide. Ces compositions présentent alors l'inconvénient de provoquer, lors de leur utilisation sur les cheveux, des coulures sur le front, la nuque, le visage ou dans les yeux, qui incommodent l'utilisateur.

On recherche donc depuis de nombreuses années des formulations cosmétiques épaissies sous forme de gels. Ce type de formulation est très appréciée par le consommateur pour des raisons de facilité et de confort d'utilisation.

La forme gel correspond le plus souvent à une préoccupation pratique pour le formulateur : faciliter la prise du produit hors de son conditionnement sans perte significative, limiter la diffusion du produit à la zone locale de traitement et pouvoir l'utiliser dans des quantités suffisantes pour obtenir l'effet cosmétique recherché. Cet objectif est important pour les formulations oxydantes utilisées pour la déformation permanente des cheveux. Celles-ci doivent bien s'étaler et se répartir de façon régulière le long des fibres kératiniques et ne pas couler sur le front, la nuque, le visage ou dans les yeux.

Il est connu d'utiliser dans les procédés de déformation permanente des cheveux, en particulier dans les procédés de lissage, des compositions oxydantes épaissies. Cependant, ces supports épaissis présentent l'inconvénient de freiner la pénétration des actifs, en particulier de l'agent oxydant, et donc de limiter l'efficacité de la déformation permanente.

De plus, il est généralement difficile d'obtenir des compositions épaissies à base de peroxyde tel que le peroxyde d'hydrogène stables à la conservation en utilisant des épaississants et/ou des gélifiants hydrosolubles classiques, par exemple ceux du type polymère acrylique réticulé comme ceux vendus sous le nom CARBOPOL par la société GOODRICH. Les peroxydes sont utilisés en cosmétique sous forme de solutions aqueuses acides pour des raisons de stabilité. Ils conduisent le plus souvent en présence des gélifiants classiques à des variations sensibles de la viscosité du gel durant le stockage.

On connaît du document FR 2 818 543 une composition cosmétique oxydante gélifiée destinée au traitement des matières kératiniques, en particulier à la déformation permanente des cheveux, comprenant un polymère amphiphile comportant au moins un monomère à insaturation éthylénique à groupement sulfonique, sous forme libre ou partiellement ou totalement neutralisée et en outre au moins une partie hydrophobe.

De telles compositions ne donnent pas totalement satisfaction.

La demanderesse a découvert de manière surprenante que l'utilisation dans une composition d'un polymère épaississant cationique qui est le produit de la polymérisation d'un mélange de monomères comprenant au moins un monomère vinylique substitué par au moins un groupe amine, au moins un monomère vinylique non ionique hydrophobe, au moins un monomère vinylique associatif, et au moins un monomère tensioactif vinylique semi-hydrophobe, permettait d'obtenir des compositions qui ne coulent pas et restent bien localisées sur la zone d'application, tout en s'étalant bien et se répartissant de façon régulière le long des fibres kératiniques, et qui, notamment dans un procédé de lissage, ne freinent pas la pénétration des actifs.

La demanderesse a également découvert que l'utilisation d'un tel polymère épaississant cationique dans une composition oxydante contenant de préférence comme agent oxydant du peroxyde d'hydrogène ou un composé oxydant susceptible de libérer du peroxyde d'hydrogène permettait d'obtenir des compositions stables au stockage.

La présente invention a donc pour objet un procédé de déformation permanente des fibres kératiniques, en particulier des cheveux, comprenant :
- une étape d'application sur les fibres kératiniques d'une composition réductrice, pour réduire les liaisons disulfure de la kératine, les fibres kératiniques étant mises en forme avant, pendant ou après ladite application, puis
- une étape de fixation par oxydation, pour reformer lesdites liaisons, par application sur les fibres kératiniques d'une composition oxydante contenant au moins un agent oxydant et au moins un polymère épaississant cationique qui est le produit de la polymérisation d'un mélange de monomères comprenant :
   a) au moins un monomère vinylique substitué par au moins un groupe amine,
   b) au moins un monomère vinylique non ionique hydrophobe,
   c) au moins un monomère vinylique associatif, et
   d) au moins un monomère tensioactif vinylique semi-hydrophobe.

La composition réductrice utilisée dans le procédé selon l'invention comprend généralement, dans un milieu cosmétiquement acceptable, au moins un agent réducteur choisi parmi les agents réducteurs de formule

H(X')_{q}(R')ᵣ

dans laquelle X' représente P, S ou SO₂, q vaut 1, r vaut 1 ou 2 et R' est un radical (C₁-C₂₀)hydrocarboné, linéaire, ramifié, saturé insaturé, éventuellement interrompu par un hétéroatome, et comportant éventuellement des substituants choisis parmi un groupement hydroxy, un groupement halogéné, un groupement amine ou un groupement carboxy, un groupement ((C₁-C₃₀) alcoxy)carbonyle, un groupement amido, un groupement ((C₁-C₃₀)alkyl)amino carbonyle, un groupement (C₁-C₃₀)acyle) amino, un groupement mono ou dialkylamino, un groupement mono ou dihydroxylamino,
ou l'un de ses sels en combinaison avec une base.

De préférence, le ou les agents réducteurs sont choisis parmi l'acide thioglycolique, l'acide thiolactique, le monothioglycolate de glycérol, la cystéamine, la N-acétyl-cystéamine, la N-propionyl-cystéamine, la cystéine, la N-acétyl-cystéine, l'acide thiomalique, la panthétéine, l'acide 2,3-dimercaptosuccinique, les N-(mercaptoalkyl)-ω-hydroxyalkylamides, les N-mono ou N,N-dialkylmercapto-4-butyramides, les aminomercapto-alkylamides, les dérivés des acides N-(mercaptoalkyl)succinamiques et des N-(mercaptoalkyl)succinimides, les alkylamino mercaptoalkylamides, le mélange azéotrope de thioglyconate de 2-hydroxypropyle et de thioglycolate de (2-hydroxy-1-méthyl)éthyle, les mercaptoalkylaminoamides, les N-mercapto-alkylalcanediamides et les dérivés d'acide formamidine sulfinique, et leurs sels.

Le ou les agents réducteurs représentent généralement de 0,05 à 30%, de préférence de 1 à 20% en poids par rapport au poids total de la composition réductrice.

Dans le but d'améliorer les propriétés cosmétiques des fibres capillaires ou encore d'atténuer ou d'éviter leur dégradation, la composition réductrice utilisée dans le procédé selon l'invention peut également comprendre un ou plusieurs actifs cosmétiques.

Ce ou ces actifs sont généralement choisis parmi les silicones, linéaires ou cycliques, les polymères cationiques, anioniques, non ioniques ou amphotères, les peptides et leurs dérivés, les hydrolysats de protéines, les cires, les agents de gonflement et de pénétration, les agents tensioactifs anioniques, cationiques, non ioniques, amphotères ou zwittérioniques, les agents antichute, les agents antipelliculaires, les épaississants naturels ou synthétiques, les agents de suspension, les agents chélatants, les agents opacifiants, les filtres solaires, les vitamines ou provitamines, les huiles minérales, végétales ou synthétiques, ainsi que les parfums et les conservateurs, et leurs mélanges.

Comme expliqué précédemment, la composition oxydante utilisée dans le procédé selon l'invention contient au moins un polymère épaississant cationique susceptible d'être obtenu par polymérisation d'un mélange de monomères spécifique.

Le ou les polymères épaississants cationiques utilisés dans la composition oxydante du procédé selon l'invention, et leur procédé de fabrication, sont décrits dans la demande internationale WO 2004/024779.

Selon un mode de réalisation préféré, le mélange de monomères comprend, par rapport au poids total du mélange de monomères :
a) de 10 à 70 % en poids du ou des monomères vinyliques substitués par au moins un groupe amine,
b) de 20 à 80 % en poids du ou des monomères vinyliques non ioniques hydrophobes,
c) de 0,001 à 25 % en poids du ou des monomères vinyliques associatifs,
d) de 0 à 25 % en poids du ou des monomères tensioactifs vinyliques semi-hydrophobes,
e) de 0 à 10 % en poids d'au moins un monomère vinylique non ionique hydroxylé,
f) de 0 à 5% en poids d'au moins un monomère réticulant,
g) de 0 à 10 % en poids d'au moins un agent de transfert de chaîne, et
h) de 0 à 2 % en poids d'au moins un stabilisateur polymérique.

Par monomère vinylique, on entend au sens de la présente invention un monomère comprenant au moins un groupe R₀CH=C(R₀)- , ou chaque R₀ est indépendamment H, un alkyle en C₁-C₃₀, -C(O)OH ou C(O)OR₀', Ro' étant un alkyle en C₁-C₃₀.

Ainsi, par exemple, au sens de la présente invention, les (méth)acrylates et les (méth)acrylamides sont des monomères vinyliques.

De façon encore plus préférée, le mélange de monomères comprend, par rapport au poids total du mélange de monomères :
a) de 20 à 60 % en poids du ou des monomères vinyliques substitués par au moins un groupe amine,
b) de 20 à 70 % en poids du ou des monomères vinyliques non ioniques hydrophobes,
c) de 0,01 à 15 % en poids du ou des monomères vinyliques associatifs,
d) de 0,1 à 10 % en poids du ou des monomères tensioactifs vinyliques semi-hydrophobes,
e) de 0,01 à 10 % en poids du ou des monomères vinyliques non ioniques hydroxylés,
f) de 0,001 à 5 % en poids du ou des monomères réticulant, et
g) de 0,001 à 10 % en poids du ou des agents de transfert de chaîne,
h) de 0 à 2 % en poids du ou des stabilisateurs polymériques.

Lorsqu'il(s) est (sont) présent(s) dans le mélange de monomères, le ou les monomères vinyliques non ioniques hydroxylés représentent de manière encore plus préférentielle 1 à 5%, en poids du poids total du mélange de monomères.

Lorsqu'il(s) est (sont) présent(s) dans le mélange de monomères, le ou les monomères réticulant représentent de préférence de 0,001 à 5% en poids du poids total du mélange de monomères.

Lorsqu'il(s) est (sont) présent(s) dans le mélange de monomères, le ou les agents de transfert de chaîne représentent de préférence de 0,1% à 10 % en poids, mieux de 0,1 % à 5 % en poids, du poids total du mélange de monomères.

Comme expliqué précédemment, le mélange de monomères permettant la préparation du polymère cationique épaississant utilisé dans le procédé selon l'invention comprend au moins un monomère vinylique substitué par au moins un groupe amine.

Les monomères vinyliques substitués par au moins un groupe amine utilisables pour la préparation du polymère cationique utilisé dans le procédé selon l'invention sont des monomères à insaturation éthylénique, basiques et polymérisables. Les groupes amines peuvent dériver de groupes alkyles mono, di- ou polyaminés, ou bien de groupes hétéroaromatiques contenant un atome d'azote. Les groupes amines peuvent être des amines primaires, secondaires ou tertiaires. Ces monomères peuvent être utilisés sous la forme d'amine ou sous la forme de sel.

De préférence, le ou les monomères vinyliques substitués par au moins un groupe amine sont choisis parmi :
- les mono-(C₁-C₄)alkylamino(C₁-C₈)alkyl(méth)acrylates
- les di-(C₁-C₄)alkylamino(C₁-C₈)alkyl(méth)acrylates, de préférence les di-(C₁-C₄)alkylamino(C₁-C₆)alkyl(méth)acrylates,
- les mono-(C₁-C₄)alkylamino(C₁-C₈)alkyl(méth)acrylamides,
- les di-(C₁-C₄)alkylamino(C₁-C₈)alkyl(méth)acrylamides,
- les (méth)acrylamides hétérocycliques contenant un atome d'azote,
- les (méth)acrylates hétérocycliques contenant un atome d'azote, et leurs mélanges.

A titre de monomères vinyliques substitués par au moins un groupe amine préférés, on peut citer : les mono- ou di-(C₁-C₄)alkylamino(C₁-C₄)alkyl(méth)acrylates, tels que le 2-(N,N-diméthylamino)éthyl (méth)acrylate, le 3-(N,N-diméthylamino)propyl (méth)acrylate, le 4-(N,N-diméthylamino)butyl (méth)acrylate, le (N,N-diméthylamino)-t-butyl (méth)acrylate, le 2-(N,N-diéthylamino)éthyl (méth)acrylate, le 3-(N,N-diéthylamino)propyl (méth)acrylate, le 4-(N,N-diéthylamino)butyl (méth)acrylate, le 2-(N,N-dipropylamino)éthyl (méth)acrylate, le 3-(N,N-dipropylamino)propyl (méth)acrylate, le 4-(N,N-dipropylamino)butyl (méth)acrylates ; les mono- ou di-(C₁-C₄)alkylamino(C₁-C₄)alkyl (méth)acrylamides tels que le N'-(2-N,N-diméthylamino)éthyl (méth)acrylamide, le N'-(3-N,N-diméthylamino)propyl acrylamide ; et les (méth)acrylamides ou (méth)acrylates hétérocycliques contenant un atome d'azote tels que le N-(2-pyridyl)acrylamide, le N-(2-imidazoyl)méthacrylamide, le 2-(4-morpholinyl)éthyl méthacrylate, le 2-(4-morpholinyl)éthyl acrylate, le N-(4-morpholinyl) méthacrylamide, le N-(4-morpholinyl) acrylamide, le 2-vinyl pyridine, et le 4-vinyl pyridine.

Lorsque les monomères sont sous forme de sels, il peut s'agir de sels minéraux, tels que les sels hydrochlorure, sulfate et phosphate ; ou bien de sels d'acides organiques, tels que les sels acétate, maléate et fumarate.

Des monomères vinyliques substitués par au moins un groupe amine particulièrement préférés sont :
- le 3-(N,N-diméthylamino)propyl (méth)acrylate,
- le N'-(3-N,N-diméthylamino)propyl (méth)acrylamide,
- le 2-(N,N-diméthylamino)éthyl (méth)acrylate,
- le 2-(N,N-diéthylamino)éthyl (méth)acrylate,
- le 2-(tert-butylamino)éthyl (méth)acrylate,
- le 2-(N,N-diméthylamino)propyl (méth)acrylamide, et
- le 2-(N,N-diméthylamino)néopentyl acrylate.

Le ou les monomères vinyliques substitués par au moins un groupe amines représentent généralement de 10 à 70 % en poids, de préférence de 20 à 60 % en poids, mieux de 30 à 40 % en poids, par rapport au poids total du mélange de monomères.

Comme expliqué précédemment, le mélange de monomères permettant la préparation du polymère cationique épaississant utilisé dans le procédé selon l'invention comprend également au moins un monomère vinylique non ionique hydrophobe.

Le ou les monomères vinyliques non ioniques hydrophobes utilisables pour la préparation du polymère cationique utilisé dans le procédé selon l'invention sont généralement choisis parmi les composés ayant l'une ou l'autre des formules (I) ou (II) :

(I) CH₂=C(X)Z,

(II) CH₂=CH-OC(O)R;

où, dans chacune des formules (I) et (II) ;
X représente H ou un groupe méthyle ;
Z représente le groupe -C(O)OR¹,-C(O)NH₂, -C(O)NHR¹, -C(O)N(R¹)₂,-C₆H₄R¹, -C₆H₄OR¹, -C₆H₄Cl, -CN, -NHC(O)CH₃, -NHC(O)H, N-(2-pyrrolidonyle), N-caprolactamyle, -C(O)NHC(CH₃)₃, -C(O)NHCH₂CH₂-N-éthylèneurée, -SiR₃, -C(O)O(CH₂)ₓSiR₃, -C(O)NH(CH₂)ₓSiR₃, ou-(CH₂)ₓSiR₃ ;
x est un entier variant de 1 à 6 ;
chaque R est indépendamment un alkyle en C₁-C₃₀ ;
chaque R¹ est indépendamment un alkyle en C₁-C₃₀, un alkyle en C₂-C₃₀ hydroxylé, ou un alkyle en C₁-C₃₀ halogéné.

On peut citer en particulier les C₁-C₃₀ alkyl (méth)acrylates ; les C₁-C₃₀ alkyl (méth)acrylamides ; le styrène, les styrènes substitués tels que le vinyl toluène (le 2-méthyl styrène), le butyl styrène, l'isopropyl styrène, le para-chloro styrène ; les esters vinyliques tels que l'acétate de vinyle, le butyrate de vinyle, le caprolate de vinyle, le pivalate de vinyle et le néodécanoate de vinyle, les nitriles insaturés tels que le (méth)acrylonitrile et l'acrylonitrile ; et les silanes insaturés tels que le triméthylvinylsilane, le diméthyléthylvinylsilane, l'allyldiméthylphénylsilane, l'allyltriméthylsilane, le 3-acrylamidopropyltriméthylsilane, le 3-triméthylsilylpropyl méthacrylate.

De préférence, le ou les monomères vinyliques non ioniques hydrophobes sont choisis parmi les C₁-C₃₀ alkyl esters d'acide acrylique, les C₁-C₃₀ alkyl esters de l'acide méthacrylique, et leurs mélanges, tels que l'acrylate d'éthyle, le méthacrylate de méthyle, le 3,3,5-triméthylcyclohexyle, et leurs mélanges.

Le ou les monomères vinyliques non ioniques hydrophobes représentent généralement de 20 à 80% en poids, de préférence de 20 à 70% en poids, mieux de 50 à 65 % en poids, par rapport au poids total du mélange de monomères.

Comme expliqué précédemment, le mélange de monomères permettant la préparation du polymère cationique épaississant utilisé dans le procédé selon l'invention comprend également au moins un monomère vinylique associatif.

Le ou les monomères vinyliques associatifs utilisables pour la préparation du polymère cationique utilisé selon l'invention sont généralement choisis parmi des composés ayant une extrémité (i) à insaturation(s) éthylénique(s) pour la polymérisation par addition avec d'autres monomères du système ; une portion centrale (ii) polyoxyalkylène pour conférer des propriétés hydrophiles sélectives aux polymères, et une extrémité (iii) hydrophobe pour conférer des propriétés hydrophobes sélectives aux polymères.

L'extrémité (i) à insaturation(s) éthylénique(s) du ou des monomères vinyliques associatifs est de préférence dérivée d'un acide ou d'un anhydride mono ou di-carboxylique à insaturation(s) α, β-éthylénique(s), de préférence un acide ou un anhydride mono ou di-carboxylique en C₃ ou C₄. De façon alternative, l'extrémité (i) du monomère associatif peut être dérivée d'un allyl éther ou d'un vinyl éther ; d'un monomère non ionique uréthane substitué par un groupe vinyle, tel que divulgué dans le brevet US redélivré n°33,156 ou dans le brevet US 5,294,692 ; ou d'un produit de réaction urée substituée par un groupe vinyle, tel que divulgué dans le brevet US 5,011,978.

La portion centrale (ii) du ou des monomères vinyliques associatifs est de préférence un segment polyoxyalkylène comprenant 5 à 250, de préférence encore 10 à 120, et mieux 15 à 60 unités oxydes d'alkylène en C₂-C₇. Des portions centrales (ii) préférées sont les segments polyoxyéthylène, polyoxypropylène, et polyoxybutylène comprenant 5 à 150, de préférence 10 à 100, et mieux 15 à 60 unités oxydes d'éthylène, de propylène ou de butylène, et des séquences aléatoires ou non aléatoires d'unités oxydes d'éthylène, oxydes de propylène ou oxydes de butylène.

L'extrémité (iii) hydrophobe du ou des monomères associatifs est de préférence un fragment hydrocarboné appartenant à l'une des classes hydrocarbonées suivantes : un alkyle linéaire en C₈-C₄₀, un alkyle en C₂-C₄₀ substitué par un groupe aryle, un phényle substitué par un groupe alkyle en C₂-C₄₀, un alkyle ramifié en C₈-C₄₀, un alkyle carboxylique en C₈-C₄₀, et un ester complexe en C₈-C₈₀.

Des exemples d'extrémités (iii) hydrophobes du ou des monomères associatifs sont des groupes alkyles linéaires ou ramifiés ayant de 8 à 40 atomes de carbone, tels que les groupes capryle (C₈), isooctyle (C₈ ramifié), décyle (C₁₀), lauryle (C₁₂), myristyle (C₁₄), cétyle (C₁₆), cétéaryle (C₁₆-C₁₈), stéaryle (C₁₈), isostéaryle (C₁₈ ramifié), arachidyle (C₂₀), béhényle (C₂₂), lignocéryle (C₂₄), cérotyle (C₂₆), montanyle (C₂₈), mélyssile (C₃₀) et laccéryle (C₃₂).

Des exemples de groupes alkyles linéaires ou ramifiés ayant 8 à 40 atomes de carbone et dérivés d'une source naturelle sont notamment les groupes alkyles dérivés de l'huile d'arachide hydrogénée, d'huile de soja et d'huile de canola (à prédominance C₁₈), l'huile de suif hydrogénée en C₁₆-C₁₈ ; et les terpénols hydrogénés en C₁₀-C₃₀, tels que le géraniol hydrogéné (en C₁₀ ramifié), le farnesol hydrogéné (C₁₅ ramifié), le phytol hydrogéné (C₂₀ ramifié).

Des exemples de phényles substitués par un alkyle en C₂-C₄₀ sont l'octylphényle, le nonylphényle, le décylphényle, le dodécylphényle, l'hexadécylphényle, l'octadécylphényle, l'isooctylphényle et le sec-butylphényle.

Des groupes alkyles carboxyliques en C₈-C₄₀ peuvent être par exemple les groupes dérivés des stérols d'origine animale, tels que le cholestérol, le lanostérol, le 7-déhydrocholestérol ; ou bien les dérivés d'origine végétales, tels que le phytostérol, le stigmastérol, le campestérol ; ou bien les dérivés issus de microorganismes, tels que l'ergostérol, le mycrostérol. D'autres groupes hydrophobes d'alkyles carboxyliques utilisables dans la présente invention sont par exemple le cyclooctyle, le cyclododécyle, l'adamantyle, le décahydronaphthyle, et les groupes dérivés de matériaux carboxyliques naturels tels que le pinène, le rétinol hydrogéné, le camphre et l'alcool d'isobornyle.

Les groupes alkyles en C₂-C₄₀ substitués par un groupe aryle peuvent être par exemple le styryle (par exemple, le 2-phényléthyle), le distyryle (par exemple, le 2,4-diphénybutyle), le tristyryle (par exemple le 2,4,6-triphénylhexyle), le 4-phénylbutyle, le 2-méthyl-2-phényléthyle, le tristyrylphénolyle.

A titre d'esters complexes en C₈-C₄₀ utilisables comme extrémité (iii), on peut citer l'huile de ricin hydrogénée (principalement le triglycéride de l'acide 12-hydroxystéarique ; les 1,2-diacyl glycérols tels que le 1,2-distéaryl glycérol, le 1,2-dipalmityl glycérol, le 1,2-dimyristyl glycérol ; les di-, tri- ou poly-esters de sucres tel que le 3,4,6-tristéaryl glucose, le 2,3-dilauryle fructose ; et les esters de sorbitane tels que ceux divulgués dans le brevet US 4,600,761.

Les monomères associatifs utilisables selon l'invention peuvent être préparés par toute méthode connue dans l'art antérieur. On peut se référer par exemple aux brevets US 4,421,902, US 4,384,096, US 4,514,552, US 4,600,761, US 4,616,074, US 5,294,692, US 5,292,843 ; US 5,770,760 et US 5,412,142.

De préférence, le ou les monomères vinyliques associatifs utilisables selon l'invention sont choisis parmi les composés de formule (III) : 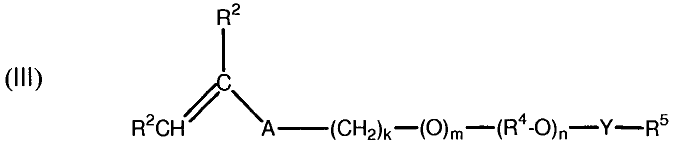 où
chaque R² est indépendamment H, un groupe méthyle, un groupe -C(O)OH, ou un groupe -C(O)OR³ ;
R³ est un alkyle en C₁-C₃₀ ;
A est un groupe -CH₂C(O)O-, -C(O)O-, -O-, CH₂O, -NHC(O)NH-, -C(O)NH-, -Ar-(CE₂)_{z}-NHC(O)O-, -Ar-(CE₂)_{z} -NHC(O)NH-, ou -CH₂CH₂-NHC(O)- ;
Ar est un groupe aryle divalent ;
E est H ou un groupe méthyle ;
z est égal à 0 ou 1 ;
k est un entier variant de 0 à 30 ;
m est égal à 0 ou 1, à la condition que lorsque k = 0, m = 0, et lorsque k varie de 1 à 30, m est égal à 1 ;
(R⁴-O)ₙ est un polyoxyalkylène, qui est un homopolymère, un copolymère aléatoire, ou un copolymère à blocs avec des unités oxyalkylène en C₂-C₄, R⁴ est C₂H₄, C₃H₆, C₄H₈, ou leurs mélanges,
n est un entier variant de 5 à 250,
Y est -R⁴O-, -R⁴NH-, -C(O)-, -C(O)NH-, R⁴NHC(O)NH-, ou -C(O)NHC(O)- ;
R⁵ est un alkyle substitué ou non choisi parmi les alkyles linéaires en C₈-C₄₀, les alkyles ramifiés en C₈-C₄₀, les alkyles carbocycliques en C₈-C₄₀, les phényles substitués par un groupe alkyle en C₂-C₄₀, les alkyles en C₂-C₄₀ substitués par un groupe aryle, les esters complexes en C₈-C₈₀,
le groupe alkyle R⁵ comprenant éventuellement un ou plusieurs substituants choisis parmi les groupes hydroxyle, alcoxyle et halogène.

De préférence, le ou les monomères vinyliques associatifs sont choisis parmi les méthacrylates polyéthoxylés de cétyle, les méthacrylates polyéthoxylés de cétéaryle, les (méth)acrylates polyéthoxylés de stéaryle, les (méth)acrylates polyéthoxylés d'arachidyle, les (méth)acrylates polyéthoxylés de béhényle, les (méth)acrylates polyéthoxylés de lauryle, les (méth)acrylates polyéthoxylés de cérotyle, les (méth)acrylates polyéthoxylés de montanyle, les (méth)acrylates polyéthoxylés de mélissyle, les (méth)acrylates polyéthoxylés de laccéryle, les (méth)acrylates phénol polyéthoxylés de tristyryle, les (méth)acrylates polyéthoxylés de l'huile de ricin hydrogénée, les (méth)acrylates polyéthoxylés de canola, les (méth)acrylates polyéthoxylés du cholestérol et leurs mélanges, ou la portion polyéthoxylée du monomère comprend de 5 à 100, de préférence de 10 à 80, et mieux de 15 à 60 unités oxydes d'éthylène.

De préférence le ou les monomères associatifs vinyliques représentent de 0,001 à 25 % en poids, de préférence de 0,01 à 15 % en poids et mieux de 0,1 à 10 % en poids du mélange de monomères.

Comme expliqué précédemment, le mélange de monomères permettant la préparation du polymère cationique épaississant utilisé dans le procédé selon l'invention comprend au moins un monomère tensioactif vinylique semi hydrophobe.

Le ou les monomères tensioactifs vinyliques semi-hydrophobes, qui contiennent une chaîne polyoxyalkylène, peuvent modérer les propriétés associatives des polymères associatifs cationiques qui les contiennent, produisant ainsi des gels aqueux ayant une très bonne texture et de très bonnes propriétés rhéologiques.

Le ou les monomères tensioactifs vinyliques semi-hydrophobes sont généralement des composés ayant deux parties :
(i) un groupe terminal insaturé pour permettre la polymérisation par addition avec les autres monomères du mélange de réaction, et
(ii) un groupe polyoxyalkylène pour atténuer les associations entre les groupes hydrophobes du polymère ou les groupes hydrophobes des autres matériaux éventuellement présents dans la composition contenant le polymère.

Un monomère tensioactif vinylique semi-hydrophobe a une structure similaire à un monomère associatif, mais a une extrémité substantiellement non hydrophobe et ainsi ne confère pas de propriété associative aux polymères.

L'extrémité fournissant l'insaturation vinylique ou éthylénique pour la polymérisation par addition est de préférence dérivée d'un acide ou d'un anhydride mono ou di-carboxylique à insaturation α, β-éthylénique, de préférence un acide mono ou di-carboxylique en C₃-C₄, ou un anhydride de cet acide. De façon alternative, l'extrémité (i) peut dériver d'un éther allylique, d'un éther vinylique ou d'un uréthane insaturé non ionique.

L'extrémité (i) insaturée polymérisable peut aussi dériver d'un acide gras insaturé en C₈-C₃₀ contenant au moins un groupe fonctionnel carboxy libre. Ce groupe en C₈-C₃₀ fait partie de l'extrémité insaturée (i) et est différent des groupes hydrophobes pendant des monomères associatifs, qui sont séparés de l'extrémité insaturée du monomère associatif par un groupe espaceur hydrophile.

La portion (ii) polyoxyalkylène comprend un segment polyoxyalkylène à chaîne longue, qui est essentiellement similaire à la portion hydrophile des monomères associatifs. Des portions polyoxyalkylène (ii) préférées incluent les unités en C₂-C₄ polyoxyéthylène, polyoxypropylène, et polyoxybutylène comprenant de 5 à 250, de préférence de 10 à 100 unités oxyalkylène. Lorsque le monomère tensioactif vinylique semi-hydrophobe comprend plus d'un type d'unité d'oxyalkylène, ces unités peuvent être disposés en séquence aléatoire, non aléatoire, ou à bloc.

De préférence, le ou les monomères tensioactifs vinyliques semi-hydrophobes sont choisis parmi les composés de formules (IV) ou (V) : 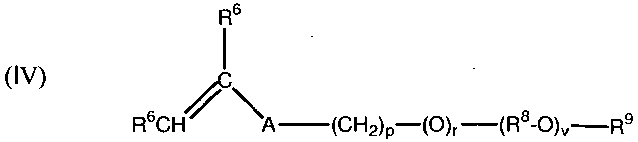 (V) D-A-(CH₂)ₚ-(O)ᵣ-(R⁸-O)ᵥ-R⁹ où, dans chaque formule (IV) ou (V),
chaque R⁶ représente indépendamment H, un alkyle en C₁-C₃₀, -C(O)OH, ou C(O)OR⁷;
R⁷est un alkyle en C₁-C₃₀ ;
A est un groupe -CH₂C(O)O-, -C(O)O-, -O-, -CH₂O, -NHC(O)NH-, -C(O)NH-, -Ar-(CE₂)_{z}-NHC(O)O-, -Ar-(CE₂)_{z} -NHC(O)NH-, ou -CH₂CH₂NHC(O)- ;
Ar est un groupe aryle divalent ;
E est H ou un groupe méthyle ;
z est égal à 0 ou 1 ;
p est un entier variant de 0 à 30 ;
r est égal à 0 ou 1, à la condition que lorsque p est égal à 0, r est égal à 0, et lorsque p varie de 1 à 30, r est égal 1,
(R₈-O)ᵥ est un polyoxyalkylène qui est homopolymère, un copolymère aléatoire, ou un copolymère à blocs avec des unités oxyalkylène en C₂-C₄, où R⁸ est C₂H₄, C₃H₆, C₄H₈, ou leurs mélanges, et v est un entier variant de 5 à 250 ;
R⁹ est H ou un alkyle en C₁-C₄ ;
D est un alkyle insaturé en C₈-C₃₀ ou un alkyle insaturé en C₈-C₃₀ substitué par un groupe carboxy.

De manière particulièrement préférée, le mélange de monomères comprend un monomère tensioactif vinylique semi-hydrophobe ayant l'une des formules suivantes :

CH₂=CH-O(CH₂)ₐO(C₃H₆O)_{b}(C₂H₄O)_{c}H ou

CH₂=CHCH₂O(C₃H₆O)_{d}(C₂H₄O)ₑH ;

où
a est égal à 2, 3, ou 4 ;
b est un entier variant de 1 à 10 ;
c est un entier variant de 5 à 50 ;
d est un entier variant de 1 à 10 ; et
e est un entier variant de 5 à 50.

Des monomères tensioactifs vinyliques semi-hydrophobes préférés sont par exemple les émulsifiants polymérisables commercialisés sous les références EMULSOGEN^{®} R109, R208, R307, RAL109, RAL208 et RAL307 par la société CLARIANT ; BX-AA-E5P5 commercialisé par la société BIMAX ; et le MAXEMUL^{®} 5010 et 5011 commercialisé par la société UNIQEMA. Les monomères particulièrement préférés sont l'EMULSOGEN^{®} R208, R307 et RAL 307.

Selon les fabricants :
l'EMULSOGEN^{®} R109 est un éther vinylique 1,4-butanediol éthoxylé/propoxylé aléatoire ayant la formule empirique :

   CH₂=CH-O(CH₂)₄O(C₃H₆O)₄(C₂H₄O)₁₀H;
l'EMULSOGEN^{®} R208 qui est un éther vinylique 1,4-butanediol éthoxylé/propoxylé aléatoire ayant la formule empirique :

   CH₂=CH-O(CH₂)₄O(C₃H₆O)₄(C₂H₄O)₂₀H;
l'EMULSOGEN^{®} R307 qui est un éther vinylique 1,4-butanediol éthoxylé/propoxylé aléatoire ayant la formule empirique :

   CH₂=CH-O(CH₂)₄O(C₃H₆O)₄(C₂H₄O)₃₀H;
l'EMULSOGEN^{®} RAL 109 qui est un éther allylique éthoxylé/propoxylé aléatoire ayant la formule empirique :

   CH₂=CHCH₂-O(C₃H₆O)₄(C₂H₄O) ₁₀H;
l'EMULSOGEN^{®} RAL 208 qui est un éther allylique éthoxylé/propoxylé aléatoire ayant la formule empirique :

   CH₂=CHCH₂-O(C₃H₆O)₄(C₂H₄O)₂₀H;
l'EMULSOGEN^{®} RAL 307 qui est un éther allylique éthoxylé/propoxylé aléatoire ayant la formule empirique :

   CH₂=CHCH₂-O(C₃H₆O)₄(C₂H₄O)₃₀H;
le MAXEMUL^{®} 5010 qui est un alcényle en C₁₂-C₁₅ carboxylé hydrophobe, éthoxylé avec 24 unités d'oxyde éthylène,
le MAXEMUL^{®} 5011 qui est un alcényle en C₁₂-C₁₅ carboxylé hydrophobe, éthoxylé avec 34 unités d'oxyde éthylène ;
et le BX-AA-E5P5 qui est un éther allylique éthoxylé/propoxylé aléatoire ayant la formule empirique :

   CH₂=CHCH₂-O(C₃H₆O)₅(C₂H₄O)₅H.

La quantité du ou des monomères tensioactifs vinyliques semi-hydrophobes utilisés dans la préparation des polymères épaississants cationiques utilisés dans le procédé selon l'invention peut varier largement et dépend, en autre, des propriétés rhéologiques finales désirées pour le polymère. Le mélange réactif de monomères contient généralement au moins 0,01 % en poids d'un ou plusieurs monomères tensioactifs vinyliques semi-hydrophobes, de préférence au moins 0,1% en poids, par rapport au poids total du mélange de monomères. Le mélange de monomères comprend de préférence au plus 25% en poids, mieux au plus 10% en poids, de monomères tensioactifs vinyliques semi-hydrophobes, par rapport au poids total du mélange de monomères.

Comme expliqué précédemment, le ou les polymères épaississants cationiques utilisés dans le procédé selon l'invention sont préparés à partir d'un mélange de monomères pouvant contenir au moins un monomère vinylique non ionique hydroxylé.

Ces monomères sont des monomères à insaturation éthylénique comprenant un ou plusieurs substituants hydroxyle.

A titre de monomères vinyliques non ioniques hydroxylés, on peut citer les (méth)acrylates d'alkyle en C₁-C₆ hydroxylés, de préférence les (méth)acrylates d'alkyle en C₁-C₄ hydroxylés, tel que le 2-hydroxyéthylméthacrylate (HEMA), le 2-hydroxyéthyl acrylate (2-HEA), le 3-hydroxypropyl acrylate ; les (méth)acrylamides d'alkyle en C₁-C₄ hydroxylés, tels que le N-(2-hydroxyéthyl) méthacrylamide, le N-(2-hydroxyéthyl) acrylamide, le N-(3-hydroxypropyl) acrylamide, le N-(2,3-dihydroxypropyl) acrylamide ; et leurs mélanges. On peut encore citer l'alcool allylique, le glycérol monoallyl éther, le 3-méthyl-3-butèn-1-ol, les précurseurs de l'alcool vinylique et leurs équivalents, tel que l'acétate de vinyle.

Lorsqu'il(s) est (sont) présent(s), le ou les monomères vinyliques non ioniques hydroxylés représentent généralement jusqu'à 10% en poids du poids total du mélange de monomères. De préférence, le ou les monomères vinyliques non ioniques hydroxylés représentent de 0,01 à 10% en poids, mieux de 1 à 8%, et encore de 1 à 5% en poids par rapport au poids total du mélange de monomères.

Comme expliqué précédemment, le ou les polymères épaississants cationiques utilisés dans le procédé selon l'invention sont préparés à partir d'un mélange de monomères qui peut comprendre un ou plusieurs monomères réticulant permettant d'introduire des ramifications et de contrôler la masse moléculaire.

Des agents réticulants poly-insaturés utilisables sont bien connus dans l'état de la technique. Des composés mono-insaturés présentant un groupe réactif capable de réticuler un copolymère formé avant, pendant ou après la polymérisation peuvent aussi être utilisés. D'autres monomères réticulant utilisables peuvent être des monomères polyfonctionnels contenant des groupes réactifs multiples tels que des groupes époxydes, isocyanates et des groupes silanes hydrolysables. De nombreux composés polyinsaturés peuvent être utilisés pour générer un réseau tri-dimensionnel partiellement ou substantiellement réticulé.

Des exemples de monomères réticulants polyinsaturés utilisables sont par exemple les monomères aromatiques polyinsaturés, tel que le divinylbenzène, le divinyl naphtylène, et le trivinylbenzène ; les monomères alicycliques polyinsaturés, tel que le 1,2,4-trivinylcyclohexane ; les esters difonctionnels de l'acide phthalique, tel que le diallyl phthalate ; les monomères aliphatiques polyinsaturés, tels que les diènes, les triènes et les tétraènes, notamment l'isoprène, le butadiène, le 1,5-hexadiène, le 1,5,9-décatriène, le 1,9-décadiène, et le 1,5 heptadiène.

D'autres monomères réticulants polyinsaturés utilisables sont par exemple les éthers polyalcényles, tels que le trialkyl pentaérythritol, le diallyl pentaérythritol, le diallyl sucrose, l'octaallyl sucrose et le triméthylolpropane diallyl éther ; les esters polyinsaturés de polyalcools ou de polyacides, tel que le 1,6-hexanediol di(méth)acrylate, le tétraméthylène tri(méth)acrylate, l'allyl acrylate, le diallyl itaconate, le diallyl fumarate, le diallyl maléate, le triméthylolpropane tri(méth)acrylate, le triméthylolpropane di(méth)acrylate et le polyéthylène glycol di(méth)acrylate, les alkylène bisacrylamides, tel que le méthylène bisacrylamide, le propylène bisacrylamide ; les dérivés hydroxylés et carboxylés du méthylène bisacrylamide, tel que le N,N'-bisméthylol méthylène bisacrylamide ; les polyéthylèneglycol di(méth)acrylates, tels que l'éthylèneglycol di(méth)acrylate, le diiéthylèneglycol di(méth)acrylate, le triéthylèneglycol di(méth)acrylate, les silanes polyinsaturés tels que le diméthyldivinylsilane, le méthyltrivinylsilane, l'allyldiméthylvinylsilane, le diallyldiméthylsilane et le tétravinylsilane, les stannanes polyinsaturés, tel que le tétraallyl étain et le diallyldiméthyl étain.

Des monomères réticulants monoinsaturés utilisables et portant un groupe réactif peuvent être les N-méthylolacrylamides ; les N-alkoxy(méth)acrylamides, où le groupe alkoxy est un groupe C₁-C₁₈ ; et les silanes hydrolysables insaturés tel que triéthoxyvinylsilane, le tris-isopropoxyvinylsilane, et le 3-triéthoxysilylpropyl méthacrylate.

Des monomères réticulants polyfonctionnels utilisables et contenant plusieurs groupes réactifs peuvent être par exemple les silanes hydrolysables tel que l'éthyltriéthoxysilane et l'éthyltriméthoxysilane ; les silanes hydrolysables époxylés, tel que le 2-(3,4-époxycyclohexyl)éthyltriéthoxysilane et le 3-glycidoxypropyltriméthyoxysilane ; les polyisocyanates, tels que le 1,4-diisocyanatobutane, le 1,6-diisocyanatohexane, le 1,4-phénylènediisocyanate, et le 4,4'-oxybis(phénylisocyanate) ; les époxides insaturés, tel que le glycidyl méthacrylate et l'allylglycidyl éther ; les polyépoxides, tel que le diglycidyl éther, le 1,2,5,6-diépoxyhexane, et l'éthylèneglycoldiglycidyl éther.

Des monomères réticulants polyinsaturés particulièrement utilisables sont les polyols éthoxylés, tels les diols, les triols et les bis-phénols, éthoxylés avec 2 à 100 moles d'oxyde d'éthylène par mole de groupe fonctionnel hydroxyle et terminés par un groupe insaturé polymérisable tel que un vinyl éther, un allyl éther, un ester acrylate ou un ester méthacrylate. De tels monomères réticulants peuvent être par exemple le diméthacrylate éthoxylé de biphénol A, le diméthacrylate éthoxylé de biphénol F, et le triméthylol propane triméthacrylate éthoxylé.

D'autres monomères réticulants éthoxylés utilisables dans la présente invention sont par exemple les agents réticulants dérivés des polyols éthoxylés divulgués dans le brevet US 6 140 435.

Des exemples particulièrement préférés de monomères réticulants sont des esters acrylates et méthacrylates de polyols ayant au moins deux groupes ester acrylate ou méthacrylate, tel que le triméthylolpropane triacrylate (TMPTA), le triméthylolpropane diméthacrylate, le triéthylène glycol diméthacrylate (TEGDMA), et le diméthacrylate de bisphénol A éthoxylé (30) (EOBDMA).

Lorsqu'il(s) est (sont) présent(s), le ou les monomères réticulants représentent de préférence au plus 5% en poids par rapport au poids du mélange de monomères. Selon un mode de réalisation préféré, les monomères réticulants sont présents à une teneur variant de 0,001 à 5% en poids, de préférence de 0,05 à 2% en poids, mieux de 0,1 à 1% en poids par rapport au poids total du mélange de monomères.

Comme expliqué précédemment, le mélange de monomères peut contenir un ou plusieurs agents de transfert de chaîne. Les agents de transferts de chaîne sont des composés bien connus de l'état de la technique.

On peut citer en particulier les composés thiolés, les composés disulfures, tels que les C₁-C₁₈ mercaptans, les acides mercaptocarboxyliques, les esters d'acides mercaptocarboxyliques, les thioesters, les C₁-C₁₈ alkyl disulfures, les aryldisulfures, les thiols polyfonctionnels ; les phosphites et hypophosphites ; les composés haloalkyl, tel que le tétrachlorure de carbone, le bromotrichlorométhane ; et les agents de transfert de chaîne insaturés, tel que l'alphaméthylstyrène.

Les thiols polyfonctionnels sont par exemple les thiols trifonctionnels, tel que le triméthylolpropane-tris-(3-mercaptopropionate), les thiols tétrafonctionnels, tel que le pentaérythritol-tétra-(3-mercaptopropionate), le pentaérythritol-tétra-(thioglycolate) et le pentaérythritol-tétra(thiolactate) ; les thiols hexafonctionnels, tel que le pentaérytritol-hexa-(thioglyconate).

De façon alternative, le ou les agents de transfert de chaîne peuvent être des agents de transfert de chaîne catalytiques qui réduisent le poids moléculaire des polymères d'addition lors de la polymérisation par radicaux libres des monomères vinyliques. On peut citer par exemple les complexes de cobalt, notamment les chélates de cobalt (II). Les agents de transfert de chaîne catalytiques peuvent souvent être utilisés à des concentrations faibles par rapport aux agents de transfert de chaîne thiolés.

De façon particulièrement préférée, on peut citer à titre d'agent de transfert de chaîne l'octyl mercaptan, le n-dodécyle mercaptan, le t-dodécyle mercaptan, l'hexadécyle mercaptan, l'octadécyle mercaptan (ODM), l'isooctyl 3-mercaptopropionate (IMP), le butyl 3-mercaptopropionate, l'acide 3-mercaptopropionique, le butyl thioglycolate, l'isooctyl thioglycolate, le dodécyle thioglycolate.

Lorsqu'il(s) est (sont) présents, le ou les agents de transferts de chaîne sont ajoutés au mélange de monomères de préférence jusqu'à 10% en poids par rapport au poids total du mélange de monomère. De préférence, le ou les agents de transfert de chaîne représentent de 0,1% à 5 % en poids par rapport au poids total de monomères.

Comme expliqué précédemment, le mélange de monomères permettant la préparation du polymère épaississant cationique utilisé dans le procédé selon l'invention peut comprendre un ou plusieurs stabilisateurs polymériques. De préférence, les polymères sont solubles dans l'eau. On peut citer par exemple les polymères synthétiques, tels que les alcools polyvinyliques, les acétates polyvinyliques partiellement hydrolysés, la polyvinylpyrrolidone, les polyacrylamides, les polyméthacrylamides, les polymères d'addition carboxylés, les polyalkyles vinyle éthers ; les polymères naturels hydrosolubles, tels que la gélatine, les peptines, les alginates, la caséine, l'amidon ; les polymères naturels modifiés, tels que la méthylcellulose, l'hydroxypropylcellulose, la carboxyméthylcellulose, les hydroxyéthylcelluloses allyliques.

Les stabilisateurs polymériques comme ceux utilisés en une quantité au plus égale à 2 % en poids par rapport au poids total de l'émulsion, de préférence en une quantité comprise 0,0001 et 1 % en poids, mieux entre 0,01 et 0,5 % en poids par rapport au poids total de l'émulsion.

Selon un mode de réalisation particulièrement préféré, le mélange de monomères permettant la préparation du polymère épaississant cationique utilisé dans le procédé selon l'invention comprend, par rapport au poids total du mélange de monomères :
a) de 20 à 50 % en poids d'au moins un monomère vinylique substitué par au moins un groupe amine choisi parmi :
   - le 3-(N,N-diméthylamino)propyl (méth)acrylate,
   - le N'-(3-N,N-diméthylamino)propyl (méth)acrylamide,
   - le 2-(N,N-diméthylamino)éthyl (méth)acrylate,
   - le 2-(N,N-diéthylamino)éthyl (méth)acrylate,
   - le 2-(tert-butylamino)éthyl (méth)acrylate,
   - le 2-(N,N-diméthylamino)propyl (méth)acrylamide, et
   - le 2-(N,N-diméthylamino)néopentyl acrylate,
b) de 50 à 65 % en poids d'au moins un monomère vinylique non ionique hydrophobe choisi parmi les C₁-C₃₀ alkyl ester d'acide acrylique, les C₁-C₃₀ alkyl ester de l'acide méthacrylique, et leurs mélanges,
c) de 0,1 à 10 % en poids d'au moins un monomère vinylique associatif choisi parmi les méthacrylates polyéthoxylés de cétyle, les méthacrylates polyéthoxylés de cétéaryle, les (méth)acrylates polyéthoxylés de stéaryle, les (méth)acrylates polyéthoxylés d'arachidyle, les (méth)acrylates polyéthoxylés de béhényle, les (méth)acrylates polyéthoxylés de lauryle, les (méth)acrylates polyéthoxylés de cérotyle, les (méth)acrylates polyéthoxylés de montanyle, les (méth)acrylates polyéthoxylés de mélissyle, les (méth)acrylates polyéthoxylés de laccéryle, les (méth)acrylates phénol polyéthoxylés de tristyryle, les (méth)acrylates polyéthoxylés de l'huile de ricin hydrogénée, les (méth)acrylates polyéthoxylés de canola, les (méth)acrylates polyéthoxylés du cholestérol et leurs mélanges,
d) de 0,1 à 10 % en poids d'au moins monomère tensioactif vinylique semi-hydrophobe ayant l'une des formules suivantes :

   CH₂=CH-O(CH₂)ₐO(C₃H₆O)_{b}(C₂H₄O)_{c}H

   ou

   CH₂=CHCH₂O(C₃H₆O)_{d}(C₂H₄O)ₑH;

   où
   a est égal à 2, 3, ou 4 ;
   b est un entier variant de 1 à 10 ;
   c est un entier variant de 5 à 50 ;
   d est un entier variant de 1 à 10 ; et
   e est un entier variant de 5 à 50.
e) jusqu'à 10 % en poids d'au moins un monomère vinylique non ionique hydroxylé,
f) jusqu'à 5% en poids d'au moins un monomère réticulant,
g) jusqu'à 10 % en poids d'au moins un agent de transfert de chaîne, et
h) jusqu'à 2 % en poids d'au moins un stabilisateur polymérique.

Le ou les polymères épaississants cationiques utilisés dans le procédé selon l'invention peuvent être préparés par des techniques de polymérisation conventionnelles, telles que la polymérisation en émulsion, comme cela est bien connu dans le domaine des polymères. La polymérisation peut être effectuée par simple procédé discontinu, par procédé par addition contrôlée, ou bien la réaction peut être initiée dans un petit réacteur puis alors la masse des monomères peut être ajoutée de façon contrôlée dans le réacteur (procédé par ensemencement). Généralement, la polymérisation est menée à une température de réaction comprise entre 20 et 80°C, même si des températures supérieures ou inférieures peuvent être utilisées. Pour faciliter l'émulsification du mélange de monomères, la polymérisation par émulsion est effectuée en présence d'un tensioactif présent en une quantité variant de 1 à 10 % en poids, de préférence de 3 à 8% en poids, mieux de 5 à 7 % en poids, par rapport au poids total de l'émulsion. Le milieu de réaction de polymérisation par émulsion comprend également un ou plusieurs initiateurs radicalaires, de préférence en une quantité variante de 0,01 à 3 % en poids par rapport au poids total du mélange de monomères. La polymérisation peut être réalisée dans un milieu aqueux ou bien hydroalcoolique à un pH neutre ou faiblement alcalin.

Dans une polymérisation typique, le mélange de monomères est ajouté sous agitation à une solution de tensioactifs émulsifiants, tel qu'un tensioactif non ionique, de préférence un éthoxylate d'alcool linéaire ou ramifié, ou un mélange de tensioactifs non ioniques et anioniques, tels que des sulfates d'alcool gras ou des alkyles sulfonates d'alcool gras, dans une quantité adaptée d'eau, dans un réacteur adapté, pour préparer l'émulsion de monomères. L'émulsion est désoxygéné au moyen de toute méthode connue, puis la réaction de polymérisation est initiée en ajoutant un catalyseur de polymérisation (initiateur) tel que le persulfate de sodium, ou tout autre catalyseur de polymérisation par addition adaptée, comme cela est bien connu dans le domaine des polymères. La réaction est agitée jusqu'à ce que la polymérisation soit complète, généralement pendant une durée variant de 4 heures à 16 heures. L'émulsion de monomères peut être chauffée à une température comprise entre 20 et 80°C avant l'addition de l'initiateur, si cela est souhaité. La quantité de monomères n'ayant pas réagi peut être éliminée par addition d'une quantité supplémentaire de catalyseur. L'émulsion de polymère obtenue peut être retirée du réacteur et emballée pour être stockée ou utilisée. De façon optionnelle, le pH ou d'autres caractéristiques physiques ou chimiques de l'émulsion peuvent être ajustés avant de retirer l'émulsion du réacteur. Généralement, l'émulsion produite a une teneur totale en solides qui varie entre 10 et 40 % en poids. Généralement, la quantité totale de polymères dans l'émulsion obtenue varie entre 15 et 35% en poids, en général au plus 25 % en poids.

Des tensioactifs adaptés pour faciliter la polymérisation par émulsion peuvent être des tensioactifs non ioniques, anioniques, amphotères ou cationiques, ou leurs mélanges. Le plus souvent, des tensioactifs non ioniques ou anioniques, ou leurs mélanges sont utilisés.

Tous types de tensioactifs non ioniques, anioniques, amphotères ou cationiques classiquement utilisés dans les polymérisations en l'émulsion peuvent être utilisés.

Comme expliqué plus haut, la polymérisation peut être effectuée en présence d'un ou plusieurs initiateurs de radicaux libres. Ceux-ci peuvent être choisis parmi les composés persulfates inorganiques insolubles, tels que le persulfate d'ammonium, le persulfate de potassium, le persulfate de sodium ; les peroxydes tels que le peroxyde d'hydrogène, le peroxyde de benzoyle, le peroxyde d'acétyle, et le peroxyde de lauryle ; les hydroperoxydes organiques, tels que l'hydroperoxyde de cumen et l'hydroperoxyde de t-butyle ; les peracides organiques, tel que l'acide peracétique ; et les agents producteurs de radicaux libres solubles dans l'huile, tels que 2,2'-azobisisobutyronitrile, et leurs mélanges. Les peroxydes et les peracides peuvent être éventuellement activés avec des agents réducteurs, tel que le bisulfite de sodium ou l'acide ascorbique, les métaux de transition, l'hydrazine. Des initiateurs de radicaux libres particulièrement adaptés sont les initiateurs de polymérisation azoïque solubles dans l'eau, tels que les composés 2,2'-azobis(tert-alkyl) ayant un substituant hydrosolubilisant sur le groupe alkyle. Des catalyseurs de polymérisation azoïque préférés sont les initiateurs de radicaux libres VAZO^{®}, commercialisés par la société DuPont, tel que le VAZO^{®}44 (2,2'-azobis(2-4,5-dihydroimidazolyl)propane), le VAZO^{®}56 (2,2'-azobis(2-méthylpropio-namidine)dihydrochlorure), et le VAZO^{®}68 (4,4'-azobis(acide 4-cyanovalérique)).

Parmi les polymères épaississants cationiques utilisés dans le procédé selon l'invention, on peut notamment citer le composé commercialisé par la société NOVEON sous la dénomination AQUA CC et qui correspond à la dénomination INCI POLYACRYLATE-1 CROSSPOLYMER.

Le POLYACRYLATE-1 CROSSPOLYMER est le produit de la polymérisation d'un mélange de monomères comprenant :
- un di-(C₁-C₄)alkylamino(C₁-C₆)alkylméthacrylate,
- un ou plusieurs esters simples en C₁-C₃₀ de l'acide (méth)acrylique,
- un polyéthylèneglycol (20-25) C₁₀-C₃₀ alkyl éther méthacrylate,
- un polyéthylèneglycol/polypropylèneglycol-30/5 allyl éther,
- un méthacrylate d'hydroxy (C₂-C₆) alkyle,
- un éthylèneglycol diméthacrylate.

Le ou les polymères épaississants cationiques utilisés dans le procédé selon l'invention représentent généralement de 0,01 à 30% en poids, de préférence de 0,1 à 10% en poids, et mieux de 0,5 à 3% en poids par rapport au poids de la composition oxydante.

Outre le ou les polymères épaississants cationiques décrits précédemment, la composition oxydante utilisée dans le procédé de déformation permanente des cheveux selon l'invention comprend au moins un agent oxydant. Celui est de préférence choisi parmi l'eau oxygénée, les bromates alcalins, les polythionates, les persels, tels que les perborates, les percarbonates et les persulfates.

De préférence, l'agent oxydant est l'eau oxygénée.

Le ou les agents oxydants représente généralement de 0,1 à 50 % de préférence de 0,2 à 20 %, en poids par rapport au poids total de la composition oxydante.

De préférence, lorsque l'agent oxydant est de l'eau oxygénée, la composition oxydante utilisée dans le procédé selon l'invention contient au moins un agent stabilisant de l'eau oxygénée.

On peut citer en particulier les pyrophosphates des métaux alcalins ou alcalino-terreux, tel que le pyrophosphate de tétrasodium, les stannates des métaux alcalins ou alcalino-terreux, la phénacétine ou les sels d'acides et d'oxyquinoléine, comme le sulfate d'oxyquinoléine. De manière plus avantageuse encore, on utilise au moins un stannate en association ou non à au moins un pyrophosphate.

Le ou les agents stabilisants de l'eau oxygénée représentent généralement de 0,0001% à 5% en poids et de préférence de 0,01 à 2% en poids par rapport au poids total de la composition oxydante.

Dans le but d'améliorer les propriétés cosmétiques des fibres capillaires ou encore d'atténuer ou d'éviter leur dégradation, la composition oxydante utilisée dans le procédé selon l'invention peut également comprendre un ou plusieurs actifs cosmétiques.

Ce ou ces actifs sont généralement choisis parmi les silicones, les polymères cationiques, autres que les polymères épaississants cationiques utilisés selon l'invention et décrits précédemment, anioniques, non ioniques ou amphotères, les peptides et leurs dérivés, les hydrolysats de protéines, les cires, les agents de gonflement et de pénétration, les agents tensioactifs anioniques, cationiques, non ioniques, amphotères ou zwittérioniques, les agents antichute, les agents antipelliculaires, les épaississants naturels ou synthétiques, autres que les polymères cationiques épaississants utilisés selon l'invention et décrits précédemment, les agents de suspension, les agents chélatants, les agents opacifiants, les filtres solaires, les vitamines ou provitamines, les huiles minérales, végétales ou synthétiques, ainsi que les parfums et les conservateurs, et leurs mélanges.

La composition oxydante utilisée dans le procédé selon l'invention contient également de préférence une ou plusieurs silicones.

Comme expliqué précédemment, la composition oxydante utilisée dans le procédé selon l'invention peut comprendre un ou plusieurs agents chélatants.

Les agents chélatants peuvent être choisis notamment parmi l'EDTA (acide éthylènediaminetétraacétique) et ses sels tels que l'EDTA disodique et l'EDTA dipotassique, les composés phosphatés tels que le métaphosphate de sodium, l'hexamétaphosphate de sodium, le pyrophosphate tétrapotassique, les acides phosphoniques et leurs sels tels que les sels de l'acide éthylènediaminetétraméthylènephosphonique et le tétrasodium étidronate.

Comme expliqué précédemment, la composition oxydante utilisée dans le procédé selon l'invention peut comprendre un ou plusieurs agents conservateurs. On peut utiliser tous types d'agents conservateurs classiquement utilisés dans les compositions cosmétiques. En particulier, on peut citer le salicylate de sodium.

Généralement, le pH des compositions oxydante ou réductrice varie de 1 à 13, de préférence de 1,5 à 12.

De préférence, lorsque l'agent oxydant est l'eau oxygénée (peroxyde d'hydrogène en solution aqueuse), le pH de la composition oxydante est compris entre 1,5 et 7 et encore plus préférentiellement entre 1,5 et 5.

De préférence le pH de la composition réductrice est compris entre 6 et 12, encore plus préférentiellement entre 7 et 11 et tout particulièrement entre 8 et 10.

Le pH des compositions aqueuses oxydantes ou réductrices selon l'invention peut être obtenu et/ou ajusté classiquement par ajout soit d'agents alcalins, tels que par exemple l'ammoniaque, la monoéthanolamine, la diéthanolamine, la triéthanolamine, l'isopropanolamine, la propanediamine-1,3, un carbonate ou bicarbonate alcalin ou d'ammonium, un carbonate organique tel que le carbonate de guanidine, ou bien encore un hydroxyde alcalin, tous ces composés pouvant bien entendu être pris seuls ou en mélange, soit d'agents acidifiants tels que par exemple l'acide chlorhydrique, l'acide acétique, l'acide lactique, l'acide borique.

Le véhicule des compositions réductrice et oxydante utilisées dans le procédé selon l'invention est de préférence un milieu aqueux constitué par de l'eau et peut avantageusement contenir des solvants organiques acceptables sur le plan cosmétique, dont plus particulièrement, des alcools tels que l'alcool éthylique, l'alcool isopropylique, l'alcool benzylique, et l'alcool phényléthylique, ou des polyols ou éthers de polyols tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol. Les solvants peuvent alors être présents dans des concentrations comprises entre environ 0,5 et 20% et, de préférence, entre environ 2 et 10% en poids par rapport au poids total de la composition.

De préférence, la composition oxydante du procédé selon l'invention possède une viscosité dynamique variant de 50 centipoises à 1000 poises, de préférence de 75 centipoises à 100 poises, à 25°C et à un taux de cisaillement de 1s⁻¹.

Comme expliqué précédemment, le procédé selon l'invention comprend une étape d'application d'une composition réductrice sur les fibres kératiniques, en particulier sur les cheveux, puis une étape d'application d'une composition oxydante.

Les cheveux sont mis en forme en utilisant des moyens mécaniques bien connus de l'homme de l'art.

Lorsque l'on souhaite réaliser une permanente (procédé de permanente), on utilise de préférence des bigoudis, la composition réductrice étant appliquée avant, pendant ou après les moyens de mise en forme des cheveux, et la composition oxydante de fixation étant appliquée après la composition réductrice, avec ou sans étape intermédiaire ou subséquente de rinçage ou d'application de composition intermédiaire.

De préférence, on applique la composition réductrice sur des cheveux mouillés préalablement enroulés sur des rouleaux ayant de 2 à 30 mm de diamètre. La composition peut également être appliquée au fur et à mesure de l'enroulage des cheveux. Généralement, on laisse ensuite agir la composition réductrice pendant un temps de 5 à 60 minutes, de préférence de 10 à 45 minutes, puis on rince abondamment. On applique alors, sur les cheveux enroulés, la composition oxydante permettant de reformer les liaisons disulfures de la kératine, avec généralement un temps de pose de 2 à 20 minutes. Après avoir enlevé les rouleaux, on rince abondamment la chevelure.

On peut également, après application de la composition réductrice, soumettre la chevelure à un traitement thermique par chauffage à une température comprise entre 30 et 220°C pendant tout ou partie du temps de pose. Dans la pratique, cette opération peut être conduite au moyen d'un casque de coiffure, d'un sèche cheveux, d'un dispensateur de rayons infrarouges et d'autres appareils chauffants classiques.

On peut notamment utiliser, à la fois comme moyen de chauffage et de mise en forme de la chevelure, un fer chauffant à une température comprise entre 60 et 220°C et de préférence entre 120 et 200°C, l'utilisation du fer chauffant se faisant entre l'étape d'application de la composition réductrice et l'étape de fixation.

Lorsque l'on souhaite effectuer le défrisage ou le décrêpage des cheveux (procédé de lissage), on applique sur les cheveux la composition réductrice, puis l'on soumet les cheveux à une déformation mécanique permettant de les fixer dans leur nouvelle forme, par une opération de lissage des cheveux par exemple avec un peigne à larges dents, avec le dos d'un peigne ou à la main. Après généralement un temps de pose de 5 à 60 minutes, de préférence de 10 à 45 minutes, on peut procéder à un nouveau lissage, puis on rince soigneusement et on applique la composition oxydante telle que définie ci-dessus, qu'on laisse agir généralement pendant environ 2 à 20 minutes, puis on rince abondamment les cheveux.

Le lissage des cheveux peut également être effectué, en tout ou partie, à l'aide d'un fer chauffant entre 60 et 220°C, et de préférence entre 120 et 200°C.

L'invention est illustrée par les exemples qui suivent.

### Exemple 1

On prépare une composition réductrice et une composition oxydante pour la mise en oeuvre d'un procédé de déformation permanente des cheveux selon l'invention.

La composition réductrice est une composition aqueuse contenant 6% en poids d'acide thioglycolique.

La formulation de la composition oxydante est la suivante :

| | |
|---|---|
| Peroxyde d'hydrogène | 4,8 g |
| Tétrasodium étidronate | 0,2 g |
| Pyrophosphate de tétrasodium | 0,04 g |
| Salicylate de sodium | 0,035 g |
| AQUA CC de NOVEON | 1% MA (en poids) |
| (Polyacrylate-1 cross polymer) | |
| Acide phosphorique | qs pH 3 |
| Eau déminéralisée | qsp 100 g |

La composition réductrice est appliquée sur des cheveux humides, préalablement enroulés sur bigoudis. La composition réductrice est laissée en contact avec la chevelure pendant 15 minutes. Puis on rince abondamment.

On applique alors sur les cheveux la composition oxydante. On laisse poser.

### Exemple 2

On prépare une composition réductrice et une composition oxydante pour la mise en oeuvre d'un procédé de déformation permanente des cheveux selon l'invention.

La composition réductrice est une composition aqueuse contenant 5,1% en poids d'acide thioglycolique et 2,5% en poids de dithioglycolate de diammonium.

La formulation de la composition oxydante est la suivante :

| | |
|---|---|
| Peroxyde d'hydrogène | 4,8 g |
| Tétrasodium étidronate | 0,2 g |
| Pyrophosphate de tétrasodium | 0,04 g |
| Salicylate de sodium | 0,035 g |
| AQUA CC de NOVEON | 2% MA (en poids) |
| (Polyacrylate-1 cross polymer) | |
| Salcare SC96 | 1 % MA (en poids) |
| Acide phosphorique | qs pH 3 |
| Eau déminéralisée | qsp 100 g |

La composition réductrice est appliquée sur des cheveux humides, préalablement enroulés sur bigoudis. La composition réductrice est laissée en contact avec la chevelure pendant 15 minutes. Puis on rince abondamment.

On applique alors sur les cheveux la composition oxydante. On laisse poser.

## Revendications

1. Procédé de déformation permanente des fibres kératiniques, en particulier des cheveux, comprenant :
- une étape d'application sur les fibres kératiniques d'une composition réductrice, pour réduire les liaisons disulfure de la kératine, les fibres kératiniques étant mises en forme avant, pendant ou après ladite application, puis
- une étape de fixation par oxydation, pour reformer lesdites liaisons, par application sur les fibres kératiniques d'une composition oxydante contenant au moins un agent oxydant et au moins un polymère épaississant cationique qui est le produit de la polymérisation d'un mélange de monomères comprenant :
a) au moins un monomère vinylique substitué par au moins un groupe amine,
b) au moins un monomère vinylique non ionique hydrophobe,
c) au moins un monomère vinylique associatif, et
d) au moins un monomère tensioactif vinylique semi-hydrophobe.

2. Procédé selon la revendication 1 **caractérisé en ce que** la composition réductrice comprend, dans un milieu cosmétiquement acceptable, au moins un agent réducteur choisi parmi les agents réducteurs de formule
H(X')_{q}(R')ᵣ
dans laquelle X' représente P, S ou SO₂, q vaut 1, r vaut 1 ou 2 et R' est un radical (C₁-C₂₀)hydrocarboné, linéaire, ramifié, saturé insaturé, éventuellement interrompu par un hétéroatome, et comportant éventuellement des substituants choisis parmi un groupement hydroxy, un groupement halogéné, un groupement amine ou un groupement carboxy, un groupement ((C₁-C₃₀) alcoxy)carbonyle, un groupement amido, un groupement ((C₁-C₃₀)alkyl)amino carbonyle, un groupement (C₁-C₃₀)acyle) amino, un groupement mono ou dialkylamino, un groupement mono ou dihydroxylamino,
ou l'un de ses sels en combinaison avec une base.

3. Procédé selon la revendication 2 **caractérisé en ce que** le ou les agents réducteurs sont choisis parmi l'acide thioglycolique, l'acide thiolactique, le monothioglycolate de glycérol, la cystéamine, la N-acétyl-cystéamine, la N-propionyl-cystéamine, la cystéine, la N-acétyl-cystéine, l'acide thiomalique, la panthétéine, l'acide 2,3-dimercaptosuccinique, les N-(mercaptoalkyl)-ω-hydroxyalkylamides, les N-mono ou N,N-dialkylmercapto-4-butyramides, les aminomercapto-alkylamides, les dérivés des acides N-(mercaptoalkyl)succinamiques et des N-(mercaptoalkyl)succinimides, les alkylamino mercaptoalkylamides, le mélange azéotrope de thioglyconate de 2-hydroxypropyle et de thioglycolate de (2-hydroxy-1-méthyl)éthyle, les mercaptoalkylaminoamides, les N-mercapto-alkylalcanediamides et les dérivés d'acide formamidine sulfinique, et leurs sels.

4. Procédé selon la revendication 2 ou 3 **caractérisé en ce que** le ou les agents réducteurs représentent de 0,05 à 30%, de préférence de 1 à 20% en poids par rapport au poids total de la composition réductrice.

5. Procédé selon l'une quelconque des revendications précédentes **caractérisé en ce que** ledit mélange de monomères comprend, par rapport au poids total du mélange de monomères :
a) de 10 à 70 % en poids du ou des monomères vinyliques substitués par au moins un groupe amine,
b) de 20 à 80 % en poids du ou des monomères vinyliques non ioniques hydrophobes,
c) de 0,001 à 25 % en poids du ou des monomères vinyliques associatifs,
d) de 0,01 à 25 % en poids du ou des monomères tensioactifs vinyliques semi-hydrophobes,
e) de 0 à 10 % en poids d'au moins un monomère vinylique non ionique hydroxylé,
f) de 0 à 5% en poids d'au moins un monomère réticulant,
g) de 0 à 10 % en poids d'au moins un agent de transfert de chaîne, et
h) de 0 à 2 % en poids d'au moins un stabilisateur polymérique.

6. Procédé selon la revendication 5 **caractérisé en ce que** le mélange de monomères comprend, par rapport au poids total du mélange de monomères :
a) de 20 à 60 % en poids du ou des monomères vinyliques substitués par au moins un groupe amine,
b) de 20 à 70 % en poids du ou des monomères vinyliques non ioniques hydrophobes,
c) de 0,01 à 15 % en poids du ou des monomères vinyliques associatifs,
d) de 0,1 à 10 % en poids du ou des monomères tensioactifs vinyliques semi-hydrophobes,
e) de 0,01 à 10 % en poids du ou des monomères vinyliques non ioniques hydroxylés,
f) de 0,001 à 5 % en poids du ou des monomères réticulant, et
g) de 0,001 à 10% en poids du ou des agents de transfert de chaîne,
h) de 0 à 2 % en poids du ou des stabilisateurs polymériques.

7. Procédé selon la revendication 5 ou 6, **caractérisé en ce que** le mélange de monomères comprend de 1 à 5 % en poids, par rapport au poids total du mélange de monomères, du ou des monomères vinyliques non ioniques hydroxylés.

8. Procédé selon l'une quelconque des revendications 5 à 7 **caractérisé en ce que** le mélange de monomères comprend de 0,001 à 5 % en poids par rapport au poids total du mélange de monomères, du ou des monomères réticulant.

9. Procédé selon l'une quelconque des revendications 5 à 8, **caractérisé en ce que** le mélange de monomères comprend de 0,1 % à 5 % en poids, par rapport au poids total du mélange de monomères, du ou des agents de transfert de chaîne.

10. Procédé selon l'une quelconque des revendications précédentes, **caractérisé en ce que** le ou les monomères vinyliques substitués par au moins un groupe amine sont choisis parmi :
- les mono-(C₁-C₄)alkylamino(C₁-C₈)alkyl(méth)acrylates,
- les di-(C₁-C₄)alkylamino(C₁-C₈)alkyl(méth)acrylates, de préférence les di-(C₁-C₄)alkylamino(C₁-C₆)alkyl(méth)acrylates,
- les mono-(C₁-C₄)a)kylamino(C₁-C₈)alkyl(méth)acrylamides,
- les di-(C₁-C₄)alkylamino(C₁-C₈)alkyl(méth)acrylamides,
- les (méth)acrylamides hétérocycliques contenant un atome d'azote,
- les (méth)acrylates hétérocycliques contenant un atome d'azote, et leurs mélanges.

11. Procédé selon la revendication 10 **caractérisé en ce que** le ou les monomères vinyliques substitués par au moins un groupe amine sont choisis parmi :
- le 3-(N,N-diméthylamino)propyl (méth)acrylate,
- le N'-(3-N,N-diméthylamino)propyl (méth)acrylamide,
- le 2-(N,N-diméthylamino)éthyl (méth)acrylate,
- le 2-(N,N-diéthylamino)éthyl (méth)acrylate,
- le 2-(tert-butylamino)éthyl (méth)acrylate,
- le 2-(N,N-diméthylamino)propyl (méth)acrylamide, et
- le 2-(N,N-diméthylamino)néopentyl acrylate.

12. Procédé selon l'une quelconque des revendications précédentes, **caractérisé en ce que** le ou les monomères vinyliques non ioniques hydrophobes sont choisis parmi les composés ayant l'une ou l'autre des formules (I) ou (II) :
(I) CH₂=C(X)Z,
(II) CH₂=CH-OC(O)R;
où, dans chacune des formules (I) et (II) ;
X représente H ou un groupe méthyle ;
Z représente le groupe -C(O)OR¹,-C(O)NH₂, -C(O)NHR¹, -C(O)N(R¹)₂, - C₆H₄R¹, -C₆H₄OR¹, -C₆H₄Cl, -CN, -NHC(O)CH₃, -NHC(O)H, N-(2-pyrrolidonyle), N-caprolactamyle, -C(O)NHC(CH₃)₃, -C(O)NHCH₂CH₂-N-éthylèneurée, -SiR₃, -C(O)O(CH₂)ₓSiR₃, -C(O)NH(CH₂)ₓSiR₃, ou-(CH₂)ₓSiR₃ ;
x est un entier variant de 1 à 6 ;
chaque R est indépendamment un alkyle en C₁-C₃₀ ;
chaque R¹ est indépendamment un alkyle en C₁-C₃₀, un alkyle en C₂-C₃₀ hydroxylé, ou un alkyle en C₁-C₃₀ halogéné.

13. Procédé selon la revendication 12 **caractérisé en ce que** le ou les monomères vinyliques non ioniques hydrophobes sont choisis parmi les C₁-C₃₀ alkyl esters de l'acide acrylique, les C₁-C₃₀ alkyl esters de l'acide méthacrylique, et leurs mélanges.

14. Procédé selon l'une quelconque des revendications précédentes **caractérisé en ce que** le ou les monomères vinyliques associatifs sont choisis parmi les composés de formule (III) : 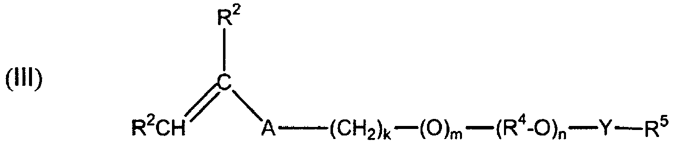 où
chaque R² est indépendamment H, un groupe méthyle, un groupe
-C(O)OH, ou un groupe -C(O)OR³;
R³ est un alkyle en C₁-C₃₀ ;
A est un groupe -CH₂C(O)O-, -C(O)O-, -O-, CH₂O, -NHC(O)NH-, -C(O)NH-, -Ar-(CE₂)_{z}-NHC(O)O-, -Ar-(CE₂)_{z} -NHC(O)NH-, ou -CH₂CH₂-NHC(O)- ;
Ar est un groupe aryle divalent ;
E est H ou un groupe méthyle ;
z est égal à 0 ou 1 ;
k est un entier variant de 0 à 30 ;
m est égal à 0 ou 1, à la condition que lorsque k = 0, m = 0, et lorsque k varie de 1 à 30, m est égal à 1 ;
(R⁴-O)ₙ est un polyoxyalkylène, qui est un homopolymère, un copolymère aléatoire, ou un copolymère à blocs avec des unités oxyalkylène en C₂-C₄, R⁴ est C₂H₄, C₃H₆, C₄H₈, ou leurs mélanges,
n est un entier variant de 5 à 250,
Y est -R⁴O-, -R⁴NH-, -C(O)-, -C(O)NH-, R⁴NHC(O)NH-, ou -C(O)NHC(O)- ;
R⁵ est un alkyle substitué ou non choisi parmi les alkyles linéaires en C₈-C₄₀, les alkyles ramifiés en C₈-C₄₀, les alkyles carbocycliques en C₈-C₄₀, les phényles substitués par un groupe alkyle en C₂-C₄₀, les alkyles en C₂-C₄₀ substitués par un groupe aryle, les esters complexes en C₈-C₈₀,
le groupe alkyle R⁵ comprenant éventuellement un ou plusieurs substituants choisis parmi les groupes hydroxyle, alcoxyle et halogène.

15. Procédé selon la revendication 14, **caractérisé en ce que** le ou les monomères vinyliques associatifs sont choisis parmi les méthacrylates polyéthoxylés de cétyle, les méthacrylates polyéthoxylés de cétéaryle, les (méth)acrylates polyéthoxylés de stéaryle, les (méth)acrylates polyéthoxylés d'arachidyle, les (méth)acrylates polyéthoxylés de béhényle, les (méth)acrylates polyéthoxylés de lauryle, les (méth)acrylates polyéthoxylés de cérotyle, les (méth)acrylates polyéthoxylés de montanyle, les (méth)acrylates polyéthoxylés de mélissyle, les (méth)acrylates polyéthoxylés de laccéryle, les (méth)acrylates phénol polyéthoxylés de tristyryle, les (méth)acrylates polyéthoxylés de l'huile de ricin hydrogénée, les (méth)acrylates polyéthoxylés de canola, les (méth)acrylates polyéthoxylés du cholestérol et leurs mélanges.

16. Procédé selon l'une quelconque des revendications précédentes, **caractérisé en ce que** le ou les monomères tensioactifs vinyliques semi-hydrophobes comprennent un groupe terminal insaturé polymérisable et un groupe polyoxyalkylène lié de façon covalente au groupe terminal.

17. Procédé selon la revendication 16 **caractérisé en ce que** le groupe polyoxyalkylène est un homopolymère, un copolymère aléatoire, ou un copolymère à blocs comprenant de 5 à 250 unités oxyalkylène en C₂-C₄.

18. Procédé selon la revendication 16 ou 17 **caractérisé en ce que** le ou les monomères tensioactifs vinyliques semi-hydrophobes sont choisis parmi les composés de formules (IV) ou (V) : 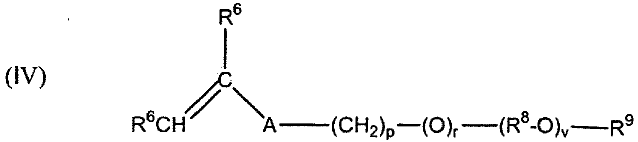
(V) D-A-(CH₂)ₚ₋(O)ᵣ-(R⁸-O)ᵥ-R⁹
où, dans chaque formule (IV) ou (V),
chaque R⁶ représente indépendamment H, un alkyle en C₁-C₃₀, -C(O)OH, ou C(O)OR⁷;
R⁷ est un alkyle en C₁-C₃₀ ;
A est un groupe -CH₂C(O)O-, -C(O)O-, -O-, -CH₂O, -NHC(O)NH-, -C(O)NH-, -Ar-(CE₂)_{z}-NHC(O)O-, -Ar-(CE₂)_{z}-NHC(O)NH-, ou -CH₂CH₂NHC(O)-;
Ar est un groupe aryle divalent ;
E est H ou un groupe méthyle ;
z est égal à 0 ou 1 ;
p est un entier variant de 0 à 30 ;
r est égal à 0 ou 1, à la condition que lorsque p est égal à 0, r est égal à 0, et lorsque p varie de 1 à 30, r est égal 1,
(R₈-O)ᵥ est un polyoxyalkylène qui est homopolymère, un copolymère aléatoire, ou un copolymère à blocs avec des unités oxyalkylène en C₂-C₄, où R⁸ est C₂H₄, C₃H₆, C₄H₈, ou leurs mélanges, et v est un entier variant de 5 à 250;
R⁹ est H ou un alkyle en C₁-C₄ ;
D est un alkyle insaturé en C₈-C₃₀ ou un alkyle insaturé en C₈-C₃₀ substitué par un groupe carboxy.

19. Procédé selon l'une quelconque des revendications 16 à 18 **caractérisé en ce que** le mélange de monomères comprend un monomère tensioactif vinylique semi-hydrophobe ayant l'une des formules suivantes :
CH₂=CH-O(CH₂)ₐO(C₃H₆O)_{b}(C₂H₄O)_{c}H
ou
CH₂=CHCH₂O(C₃H₆O)_{d}(C₂H₄O)ₑH;
où
a est égal à 2, 3, ou 4 ;
b est un entier variant de 1 à 10 ;
c est un entier variant de 5 à 50 ;
d est un entier variant de 1 à 10 ; et
e est un entier variant de 5 à 50.

20. Procédé selon l'une quelconque des revendications 5 à 19, **caractérisé en ce que** le ou les monomères vinyliques non ioniques hydroxylés sont choisis parmi les acrylates d'alkyle en C₁-C₆ hydroxylés, de préférence les acrylates d'alkyle en C₁-C₄ hydroxylés, les méthacrylates d'alkyle en C₁-C₆ hydroxylés, de préférence les méthacrylates d'alkyle en C₁-C₄ hydroxylés, les acrylamides d'alkyle en C₁-C₄ hydroxylés, les méthacrylamides d'alkyle en C₁-C₄ hydroxylés et leurs mélanges.

21. Procédé selon la revendication 20, **caractérisé en ce que** le monomère vinylique non ionique hydroxylé est le méthacrylate de 2-hydroxyéthyle.

22. Procédé selon l'une quelconque des revendications 5 à 21, **caractérisé en ce que** le monomère réticulant est un ester acrylate de polyol ayant au moins deux groupes ester acrylate, un ester méthacrylate de polyol ayant au moins deux groupes ester méthacrylate, ou une combinaison de ceux-ci.

23. Procédé selon l'une quelconque des revendications 5 à 22, **caractérisé en ce que** l'agent de transfert de chaîne est choisi parmi les composés thiolés, les composé disulfures, les phosphites, les hypophosphites, les composés haloalkyles, et leurs combinaisons.

24. Procédé selon l'une quelconque des revendications 5 à 23 **caractérisé en ce que** ledit mélange de monomères comprend, par rapport au poids total du mélange de monomères :
a) de 20 à 50 % en poids d'au moins un monomère vinylique substitué par au moins un groupe amine choisi parmi :
- le 3-(N,N-diméthylamino)propyl (méth)acrylate,
- le N'-(3-N,N-diméthylamino)propyl (méth)acrylamide,
- le 2-(N,N-diméthylamino)éthyl (méth)acrylate,
- le 2-(N,N-diéthylamino)éthyl (méth)acrylate,
- le 2-(tert-butylamino)éthyl (méth)acrylate,
- le 2-(N,N-diméthylamino)propyl (méth)acrylamide, et
- le 2-(N,N-diméthylamino)néopentyl acrylate,
b) de 50 à 65 % en poids d'au moins un monomère vinylique non ionique hydrophobe choisi parmi les C₁-C₃₀ alkyl ester d'acide acrylique, les C₁-C₃₀ alkyl ester de l'acide méthacrylique, et leurs mélanges,
c) de 0,1 à 10 % en poids d'au moins un monomère vinylique associatif choisi parmi les méthacrylates polyéthoxylés de cétyle, les méthacrylates polyéthoxylés de cétéaryle, les (méth)acrylates polyéthoxylés de stéaryle, les (méth)acrylates polyéthoxylés d'arachidyle, les (méth)acrylates polyéthoxylés de béhényle, les (méth)acrylates polyéthoxylés de lauryle, les (méth)acrylates polyéthoxylés de cérotyle, les (méth)acrylates polyéthoxylés de montanyle, les (méth)acrylates polyéthoxylés de mélissyle, les (méth)acrylates polyéthoxylés de laccéryle, les (méth)acrylates phénol polyéthoxylés de tristyryle, les (méth)acrylates polyéthoxylés de l'huile de ricin hydrogénée, les (méth)acrylates polyéthoxylés de canola, les (méth)acrylates polyéthoxylés du cholestérol et leurs mélanges,
d) de 0,1 à 10 % en poids d'au moins monomère tensioactif vinylique semi-hydrophobe ayant l'une des formules suivantes :
CH₂=CH-O(CH₂)ₐO(C₃H₆O)_{b}(C₂H₄O)_{c}H
ou
CH₂=CHCH₂O(C₃H₆O)_{d}(C₂H₄O)ₑH;
où
a est égal à 2, 3, ou 4 ;
b est un entier variant de 1 à 10 ;
c est un entier variant de 5 à 50 ;
d est un entier variant de 1 à 10 ; et
e est un entier variant de 5 à 50.
e) jusqu'à 10 % en poids d'au moins un monomère vinylique non ionique hydroxylé,
f) jusqu'à 5% en poids d'au moins un monomère réticulant,
g) jusqu'à 10 % en poids d'au moins un agent de transfert de chaîne, et
h) jusqu'à 2 % en poids d'au moins un stabilisateur polymérique.

25. Procédé selon l'une quelconque des revendications précédentes **caractérisé en ce que** ledit mélange de monomères comprend :
- un di-(C₁-C₄)alkylamino(C₁-C₆)alkylméthacrylate,
- un ou plusieurs esters simples en C₁-C₃₀ de l'acide (méth)acrylique,
- un polyéthylèneglycol (20-25) C₁₀-C₃₀ alkyl éther méthacrylate,
- un polyéthylèneglycol/polypropylèneglycol-30/5 allyl éther,
- un méthacrylate d'hydroxy (C₂-C₆) alkyle,
- un éthylèneglycol diméthacrylate.

26. Procédé selon l'une quelconque des revendications précédentes **caractérisé en ce que** le ou les polymères épaississants cationiques utilisés dans le procédé selon l'invention représentent de 0,01 à 30% en poids, de préférence de 0,1 à 10% en poids, et mieux de 0,5 à 3% en poids par rapport au poids de la composition oxydante.

27. Procédé selon l'une quelconque des revendications précédentes **caractérisé en ce que** le ou lesdits agents oxydants sont choisis parmi l'eau oxygénée, les bromates alcalins, les polythionates, les persels, tels que les perborates, les percarbonates et les persulfates.

28. Procédé selon la revendication 27 **caractérisé en ce que** le ou les agents oxydants représentent généralement de 0,1 à 50 %, de préférence de 0,2 à 20 % en poids par rapport au poids total de la composition oxydante.

29. Procédé selon la revendication 27 ou 28 **caractérisé en ce que** la composition oxydante contient de l'eau oxygénée et au moins un agent stabilisant de l'eau oxygénée choisi parmi les pyrophosphates des métaux alcalins ou alcalino-terreux, tel que le pyrophosphate de tétrasodium, les stannates des métaux alcalins ou alcalino-terreux, la phénacétine ou les sels d'acides et d'oxyquinoléine, tel que le sulfate d'oxyquinoléine.

30. Procédé selon la revendication 29 **caractérisé en ce que** le ou les agents stabilisants de l'eau oxygénée représentent de 0,0001% à 5% en poids, de préférence de 0,01 à 2% en poids, par rapport au poids total de la composition oxydante.

31. Procédé selon l'une quelconque des revendications précédentes **caractérisé en ce que** la viscosité dynamique de la composition oxydante, mesurée à 25°C et à un taux de cisaillement de 1 s⁻¹, varie de 50 centipoises à 1000 poises, de préférence de 75 centipoises à 100 poises.

32. Procédé selon l'une quelconque des revendications précédentes **caractérisé en ce que** le pH de la composition oxydante varie de 1 à 13, de préférence de 1,5 à 12.

33. Procédé selon l'une quelconque des revendications précédentes **caractérisé en ce que** on laisse la composition réductrice agir pendant 5 à 60 minutes, de préférence pendant 10 à 45 minutes.

34. Procédé selon l'une quelconque des revendications précédentes **caractérisé en ce qu'**il comprend, entre l'étape d'application sur les cheveux de la composition réductrice et l'étape de fixation, une étape de chauffage des cheveux avec un fer chauffant à une température comprise entre 60 et 220°C, de préférence entre 120 et 200°C.

35. Procédé selon l'une quelconque des revendications précédentes **caractérisé en ce que** on laisse agir la composition oxydante pendant 2 à 20 minutes.

## Patentansprüche

1. Verfahren zur permanenten Verformung von Keratinfasern, insbesondere des Haars, umfassend:
- einen Schritt des Aufbringens einer reduzierenden Zusammensetzung auf die Keratinfasern zur Reduktion der Disulfidbindungen des Keratins, wobei die Keratinfasern vor, während oder nach dem Aufbringen in Form gebracht werden, und dann
- einen Schritt des oxidativen Fixierens zur Wiederherstellung der Bindungen durch Aufbringen einer oxidierenden Zusammensetzung, die mindestens ein Oxidationsmittel und mindestens ein kationisches verdickendes Polymer umfasst, auf die Keratinfasern, wobei es sich bei dem kationischen verdickenden Polymer um das Produkt der Polymerisation einer Monomerenmischung, die
a) mindestens ein Vinylmonomere, das durch mindestens eine Amingruppe substituiert ist,
b) mindestens ein hydrophobes nichtionisches Vinylmonomer,
c) mindestens ein assoziatives Vinylmonomer und
d) mindestens ein semihydrophobes oberflächenaktives Vinylmonomer umfasst, handelt.

2. Verfahren nach Anspruch 1, **dadurch gekennzeichnet, dass** die reduzierende Zusammensetzung in einem kosmetisch unbedenklichen Medium mindestens ein Reduktionsmittel umfasst, das aus den Reduktionsmitteln der Formel
H(X')_{q}(R')ᵣ
wobei X' für P, S oder SO₂ steht, q gleich 1 ist, r gleich 1 oder 2 ist und R' für einen linearen, verzweigten, gesättigten ungesättigten (C₁-C₂₀)-Kohlenwasserstoffrest, der gegebenenfalls durch ein Heteroatom unterbrochen ist und gegebenenfalls Substituenten, die aus einer Hydroxygruppe, einer halogenierten Gruppe, einer Amingruppe oder einer Carboxygruppe, einer ((C₁-C₃₀) Alkoxy) carbonyl-gruppe, einer Amidogruppe, einer ((C₁-C₃₀)Alkyl)-aminocarbonylgruppe, einer ((C₁-C₃₀)Acyl)aminogruppe, einer Monoalkylamino- oder Dialkylaminogruppe, einer Monohydroxylamino- oder Dihydroxylaminogruppe ausgewählt sind, umfasst, steht,
oder einem Salz davon in Kombination mit einer Base ausgewählt ist.

3. Verfahren nach Anspruch 2, **dadurch gekennzeichnet, dass** das bzw. die Reduktionsmittel aus Thioglykolsäure, Thiomilchsäure, Glycerinmonothioglycolat, Cysteamin, N-Acetylcysteamin, N-Propionylcysteamin, Cystein, N-Acetylcystein, Thioäpfelsäure, Panthetein, 2,3-Dimercaptobernsteinsäure, N-(Mercaptoalkyl)-ω-hydroxyalkylamiden, N-Mono-oder N,N-Dialkylmercapto-4-butyramiden, Aminomercaptoalkylamiden, Derivaten von N-(Mercapto-alkyl)succinamidsäuren und N-(Mercaptoalkyl)-succinimiden, Alkylaminomercaptoalkylamiden, dem azeotropen Gemisch von 2-Hydroxypropylthioglykonat und (2-Hydroxy-1-methyl)ethylthioglykolat, Mercaptoalkylaminoamiden, N-Mercaptoalkylalkandiamiden und Formamidinsulfinsäurederivaten und Salzen davon ausgewählt ist bzw. sind.

4. Verfahren nach Anspruch 2 oder 3, **dadurch gekennzeichnet, dass** das bzw. die Reduktionsmittel 0,05 bis 30 Gew.-% und vorzugsweise 1 bis 20 Gew.-%, bezogen auf das Gesamtgewicht der reduzierenden Zusammensetzung, ausmacht bzw. ausmachen.

5. Verfahren nach einem der vorhergehenden Ansprüche, **dadurch gekennzeichnet, dass** die Monomerenmischung, bezogen auf das Gesamtgewicht der Monomerenmischung, Folgendes umfasst:
a) 10 bis 70 Gew.-% des bzw. der Vinylmonomere, die durch mindestens eine Amingruppe substituiert sind,
b) 20 bis 80 Gew.-% des bzw. der hydrophoben nichtionischen Vinylmonomere,
c) 0,001 bis 25 Gew.-% des bzw. der assoziativen Vinylmonomere,
d) 0,01 bis 25 Gew.-% des bzw. der semihydrophoben oberflächenaktiven Vinylmonomere,
e) 0 bis 10 Gew.-% mindestens eines hydroxylierten nichtionischen Vinylmonomers,
f) 0 bis 5 Gew.-% mindestens eines Vernetzungsmonomers,
g) 0 bis 10 Gew.-% mindestens eines Kettenübertragungsmittels und
h) 0 bis 2 Gew.-% mindestens eines polymeren Stabilisators.

6. Verfahren nach Anspruch 5, **dadurch gekennzeichnet, dass** die Monomerenmischung, bezogen auf das Gesamtgewicht der Monomerenmischung, Folgendes umfasst:
a) 20 bis 60 Gew.-% des bzw. der Vinylmonomere, die durch mindestens eine Amingruppe substituiert sind,
b) 20 bis 70 Gew.-% des bzw. der hydrophoben nichtionischen Vinylmonomere,
c) 0,01 bis 15 Gew.-% des bzw. der assoziativen Vinylmonomere,
d) 0,1 bis 10 Gew.-% des bzw. der semihydrophoben oberflächenaktiven Vinylmonomere,
e) 0,01 bis 10 Gew.-% des bzw. der hydroxylierten nichtionischen Vinylmonomere,
f) 0,001 bis 5 Gew.-% des bzw. der Vernetzungsmonomere,
g) 0,001 bis 10 Gew.-% des bzw. der Kettenübertragungsmittel und
h) 0 bis 2 Gew.-% des bzw. der polymeren Stabilisatoren.

7. Verfahren nach Anspruch 5 oder 6, **dadurch gekennzeichnet, dass** die Monomerenmischung 1 bis 5 Gew.-%, bezogen auf das Gesamtgewicht der Monomerenmischung, des bzw. der hydroxylierten nichtionischen Vinylmonomere umfasst.

8. Verfahren nach einem der Ansprüche 5 bis 7, **dadurch gekennzeichnet, dass** die Monomerenmischung 0,001 bis 5 Gew.-%, bezogen auf das Gesamtgewicht der Monomerenmischung, des bzw. der Vernetzungsmonomere umfasst.

9. Verfahren nach einem der Ansprüche 5 bis 8, **dadurch gekennzeichnet, dass** die Monomerenmischung 0,1 bis 5 Gew.-%, bezogen auf das Gesamtgewicht der Monomerenmischung, des bzw, der Kettenübertragungsmittel umfasst.

10. Verfahren nach einem der vorhergehenden Ansprüche, **dadurch gekennzeichnet, dass** das bzw. die Vinylmonomere, die durch mindestens eine Amingruppe substituiert sind, aus:
- Mono (C₁-C₄) alkylamino (C₁-C₈) alkyl (meth) acrylaten,
- Di (C₁-C₄) alkylamino (C₁-C₈) alkyl (meth) acrylaten, vorzugsweise Di (C₁-C₄) alkylamino (C₁-C₆) alkyl-(meth)acrylaten,
- Mono (C₁-C₄) alkylamino (C₁-C₈) alkyl (meth) acrylamiden,
- Di (C₁-C₄) alkylamino (C₁-C₈) alkyl (meth) acrylamiden,
- heterocyclischen (Meth)acrylamiden mit einem Stickstoffatom,
- heterocyclischen (Meth)acrylaten mit einem Stickstoffatom
und Mischungen davon ausgewählt ist bzw. sind.

11. Verfahren nach Anspruch 10, **dadurch gekennzeichnet, dass** das bzw. die Vinylmonomere, die durch mindestens eine Amingruppe substituiert sind, aus:
- 3-(N,N-Dimethylamino)propyl(meth)acrylat,
- N'-(3-N,N-Dimethylamino)propyl(meth)acrylamid,
- 2-(N,N-Dimethylamino)ethyl(meth)acrylat,
- 2-(N,N-Diethylamino)ethyl(meth)acrylat,
- 2-(tert-Butylamino)ethyl(meth)acrylat,
- 2-(N,N-Dimethylamino)propyl(meth)acrylamid und
- 2-(N,N-Dimethylamino)neopentylacrylat
ausgewählt ist bzw. sind.

12. Verfahren nach einem der vorhergehenden Ansprüche, **dadurch gekennzeichnet, dass** das bzw. die hydrophoben nichtionischen Vinylmonomere aus den Verbindungen mit einer der Formeln (I) oder (II):
(I) CH₂=C(X)Z,
(II) CH₂=CH-OC(O)R
ausgewählt ist bzw. sind, wobei in den Formeln (I) und (II) :
X für H oder eine Methylgruppe steht;
Z für die Gruppe -C(O)OR¹, -C(O)NH₂, -C(O)NHR¹, -C(O)N(R¹)₂, -C₆H₄R¹, -C₆H₄OR¹, -C₆H₄Cl, -CN, -NHC(O)CH₃, -NHC(0)H, N-(2-Pyrrolidonyl), N-Caprolactamyl, -C(O)NHC(CH₃)₃, -C(O)NHCH₂CH₂-N-Ethylenharnstoff, -SiR₃, -C(O)O(CH₂)ₓSiR₃, C(O)NH(CH₂)ₓSiR₃ oder -(CH₂)ₓSiR₃ steht;
x für eine ganze Zahl von 1 bis 6 steht;
R jeweils unabhängig für eine C₁-C₃₀-Alkylgruppe steht;
R¹ jeweils unabhängig für eine C₁-C₃₀-Alkylgruppe, eine C₂-C₃₀-Hydroxyalkylgruppe oder eine C₁-C₃₀-Halogenalkylgruppe steht.

13. Verfahren nach Anspruch 12, **dadurch gekennzeichnet, dass** das bzw. die hydrophoben nichtionischen Vinylmonomere aus C₁-C₃₀-Alkylestern von Acrylsäure, C₁-C₃₀-Alkylestern von Methacrylsäure und Mischungen davon ausgewählt ist bzw. sind.

14. Verfahren nach einem der vorhergehenden Ansprüche, **dadurch gekennzeichnet, dass** das bzw. die assoziativen Vinylmonomere aus den Verbindungen der Formel (III): 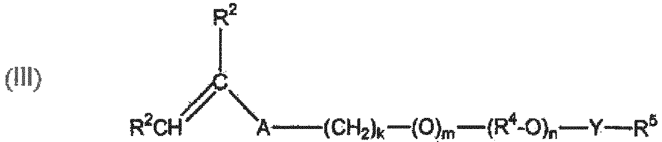 ausgewählt ist bzw. sind, wobei
R² jeweils unabhängig für H, eine Methylgruppe, eine Gruppe -C(O)OH oder eine Gruppe -C(O)OR³ steht;
R³ für eine C₁-C₃₀-Alkylgruppe steht;
A für eine Gruppe -CH₂C(O)O-, -C(O)O-, -O-, CH₂O, -NHC(O)NH-, -C(O)NH-, -Ar-(CE₂)_{z}-NHC(O)O-, -Ar-(CE₂)_{z}-NHC(O)NH- oder -CH₂CH₂-NHC(O)- steht;
Ar für eine zweiwertige Arylgruppe steht;
E für H oder eine Methylgruppe steht;
z gleich 0 oder 1 ist;
k für eine ganze Zahl im Bereich von 0 bis 30 steht;
m gleich 0 oder 1 ist, mit der Maßgabe, dass dann, wenn k = 0, m = 0, und dann, wenn k im Bereich von 1 bis 30 liegt, m gleich 1 ist;
(R⁴-O)ₙ für ein Polyoxyalkylen, das ein Homopolymer, ein statistisch aufgebautes Copolymer oder ein Blockcopolymer mit C₂-C₄-Oxyalkyleneinheiten ist, steht,
R⁴ für C₂H₄, C₃H₆, C₄H₈ oder Mischungen davon steht; n für eine ganze Zahl im Bereich von 5 bis 250 steht;
Y für -R⁴O-, -R⁴NH-, -C(O)-, -C(O)NH-, R⁴NHC(O)NH-oder -C(O)NHC(O)- steht;
R⁵ für ein gegebenenfalls substituiertes Alkyl, ausgewählt aus linearen C₈-C₄₀-Alkylgruppen, verzweigten C₈-C₄₀-Alkylgruppen, carbocyclischen C₈-C₄₀-Gruppen, durch eine C₂-C₄₀-Alkylgruppe substituierten Phenylgruppen, durch eine Arylgruppe substituierten C₂-C₄₀-Alkylgruppen und C₈-C₈₀-Komplexestern, steht,
wobei die Alkylgruppe R⁵ gegebenenfalls einen oder mehrere aus Hydroxyl-, Alkoxy- und Halogengruppen ausgewählte Substituenten umfasst.

15. Verfahren nach Anspruch 14, **dadurch gekennzeichnet, dass** das bzw. die assoziativen Vinylmonomere aus polyethoxylierten Cetylmethacrylaten, polyethoxylierten Cetearylmethacrylaten, polyethoxylierten Stearyl(meth)acrylaten, polyethoxylierten Arachidyl(meth)acrylaten, polyethoxylierten Behenyl(meth)acrylaten, polyethoxylierten Lauryl-(meth)acrylaten, polyethoxylierten Cerotyl(meth)-acrylaten, polyethoxylierten Montanyl(meth)-acrylaten, polyethoxylierten Melissyl(meth)-acrylaten, polyethoxylierten Lacceryl(meth)-acrylaten, polyethoxylierten Tristyrylphenol-(meth)acrylaten, polyethoxylierten (Meth)acrylaten von hydriertem Rizinusöl, polyethoxylierten Canola(meth)acrylaten, polyethoxylierten Cholesterin(meth)acrylaten und Mischungen davon ausgewählt ist bzw. sind.

16. Verfahren nach einem der vorhergehenden Ansprüche, **dadurch gekennzeichnet, dass** das bzw. die semihydrophoben oberflächenaktiven Vinylmonomere eine polymerisierbare ungesättigte Endgruppe und eine kovalent daran gebundene Polyoxyalkylengruppe umfasst bzw. umfassen.

17. Verfahren nach Anspruch 16, **dadurch gekennzeichnet, dass** es sich bei der Polyoxyalkylengruppe um ein Homopolymer, ein statistisch aufgebautes Copolymer oder ein Blockcopolymer mit 5 bis 250 C₂-C₄-Oxyalkyleneinheiten handelt.

18. Verfahren nach Anspruch 16 oder 17, **dadurch gekennzeichnet, dass** das bzw. die semihydrophoben oberflächenaktiven Vinylmonomere aus den Verbindungen der Formel (IV) oder (V): 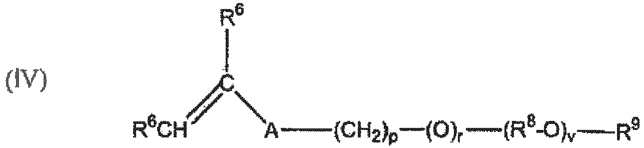
(V) D-A-CH₂)ₚ-(O)ᵣ-(R⁸-O)ᵥ-R⁹
ausgewählt ist bzw. sind, wobei in jeder Formel (IV) oder (V):
R⁶ jeweils unabhängig für H, eine C₁-C₃₀-Alkylgruppe, -C(O)OH oder C(O)OR⁷ steht;
R⁷ für eine C₁-C₃₀-Alkylgruppe steht;
A für eine Gruppe -CH₂C(O)O-, -C(O)O-, -O-, -CH₂O, -NHC(O)NH-, -C(O)NH-, -Ar-(CE₂)_{z}-NHC(O)O- , -Ar-(CE₂)_{z}-NHC(O)NH- oder -CH₂CH₂-NHC(O)- steht;
Ar für eine zweiwertige Arylgruppe steht;
E für H oder eine Methylgruppe steht;
z gleich 0 oder 1 ist;
p für eine ganze Zahl im Bereich von 0 bis 30 steht;
r gleich 0 oder 1 ist, mit der Maßgabe, dass dann, wenn p gleich 0 ist, r gleich 0 ist, und dann, wenn p im Bereich von 1 bis 30 liegt, r gleich 1 ist;
(R₈-O)ᵥ für ein Polyoxyalkylen, das ein Homopolymer, ein statistisch aufgebautes Copolymer oder ein Blockcopolymer mit C₂-C₄-Oxyalkylen-einheiten ist, steht, wobei R⁸ für C₂H₄, C₃H₆, C₄H₈ oder Mischungen davon steht und v für eine ganze Zahl im Bereich von 5 bis 250 steht;
R⁹ für H oder eine C₁-C₄-Alkylgruppe steht;
D für eine ungesättigte C₈-C₃₀-Alkylgruppe oder eine ungesättigte durch eine Carboxygruppe substituiert C₈-C₃₀-Alkylgruppe steht.

19. Verfahren nach einem der Ansprüche 16 bis 18, **dadurch gekennzeichnet, dass** die Monomerenmischung ein semihydrophobes oberflächenaktives Vinylmonomer mit einer der folgenden Formeln umfasst:
CH₂=CH-O(CH₂)ₐO(C₃H₆O)_{b}(C₂H₄0)_{c}H
oder
CH₂=CHCH₂O(C₃H₆O)_{d}(C₂H₄O)ₑH;
wobei
a gleich 2, 3 oder 4 ist;
b für eine ganze Zahl im Bereich von 1 bis 10 steht;
c für eine ganze Zahl im Bereich von 5 bis 50 steht;
d für eine ganze Zahl im Bereich von 1 bis 10 steht; und
e für eine ganze Zahl im Bereich von 5 bis 50 steht.

20. Verfahren nach einem der Ansprüche 5 bis 19, **dadurch gekennzeichnet, dass** das bzw. die hydroxylierten nichtionischen Vinylmonomere aus C₁-C₆-Hydroxyalkylacrylaten, vorzugsweise C₁-C₄-Hydroxyalkylacrylaten, C₁-C₆-Hydroxyalkylmethacrylaten, vorzugsweise C₁-C₄-Hydroxyalkylmethacrylaten, (C₁-C₄-Hydroxyalkyl)acrylamiden, (C₁-C₄-Hydroxyalkyl)methacrylamiden und Mischungen davon ausgewählt ist bzw. sind.

21. Verfahren nach Anspruch 20, **dadurch gekennzeichnet, dass** es sich bei dem hydroxylierten nichtionischen Vinylmonomer um 2-Hydroxyethylmethacrylat handelt.

22. Verfahren nach einem der Ansprüche 5 bis 21, **dadurch gekennzeichnet, dass** es sich bei dem Vernetzungsmonomer um einen Polyolacrylatester mit mindestens zwei Acrylatestergruppen, einen Polyolmethacrylatester mit mindestens zwei Methacrylatestergruppen oder eine Kombination davon handelt.

23. Verfahren nach einem der Ansprüche 5 bis 22, **dadurch gekennzeichnet, dass** das Kettenübertragungsmittel aus Thiolverbindungen, Disulfidverbindungen, Phosphiten, Hypophosphiten, Halogenalkylverbindungen und Kombinationen davon ausgewählt ist.

24. Verfahren nach einem der Ansprüche 5 bis 23, **dadurch gekennzeichnet, dass** die Monomerenmischung, bezogen auf das Gesamtgewicht der Monomerenmischung, Folgendes umfasst:
a) 20 bis 50 Gew.-% mindestens eines Vinylmonomers, das durch mindestens eine Amingruppe substituiert ist, ausgewählt aus:
- 3-(N,N-Dimethylamino)propyl(meth)acrylat,
- N'-(3-N,N-Dimethylamino)propyl(meth)acrylamid,
- 2-(N,N-Dimethylamino)ethyl(meth)acrylat,
- 2-(N,N-Diethylamino)ethyl(meth)acrylat,
- 2-(tert-Butylamino)ethyl(meth)acrylat,
- 2-(N,N-Dimethylamino)propyl(meth)acrylamid und
- 2-(N,N-Dimethylamino)neopentylacrylat,
b) 50 bis 65 Gew. -% mindestens eines hydrophoben nichtionischen Vinylmonomers, ausgewählt aus C₁-C₃₀-Alkylestern von Acrylsäure, C₁-C₃₀-Alkyl-estern von Methacrylsäure und Mischungen davon,
c) 0,1 bis 10 Gew.-% mindestens eines assoziativen Vinylmonomers, ausgewählt aus polyethoxylierten Cetylmethacrylaten, polyethoxylierten Cetearylmethacrylaten, polyethoxylierten Stearyl(meth)-acrylaten, polyethoxylierten Arachidyl(meth)-acrylaten, polyethoxylierten Behenyl(meth)-acrylaten, polyethoxylierten Lauryl(meth)-acrylaten, polyethoxylierten Cerotyl(meth)-acrylaten, polyethoxylierten Montanyl(meth)-acrylaten, polyethoxylierten Melissyl(meth)-acrylaten, polyethoxylierten Lacceryl(meth)-acrylaten, polyethoxylierten Tristyrylphenol-(meth)acrylaten, polyethoxylierten (Meth)-acrylaten von hydriertem Rizinusöl, polyethoxylierten Canola(meth)acrylaten, polyethoxylierten Cholesterin(meth)acrylaten und Mischungen davon,
d) 0,1 bis 10 Gew.-% mindestens eines semihydrophoben oberflächenaktiven Vinylmonomers mit einer der folgenden Formeln:
CH₂=CH-O(CH₂)ₐO(C₃H₆O)_{b}(C₂H₄O)_{c}H
oder
CH₂=CHCH₂O(C₃H₆O)_{d}(C₂H₄O)ₑH ;
wobei
a gleich 2, 3 oder 4 ist;
b für eine ganze Zahl im Bereich von 1 bis 10 steht;
c für eine ganze Zahl im Bereich von 5 bis 50 steht;
d für eine ganze Zahl im Bereich von 1 bis 10 steht; und
e für eine ganze Zahl im Bereich von 5 bis 50 steht,
e) bis zu 10 Gew.-% mindestens eines hydroxylierten nichtionischen Vinylmonomers,
f) bis zu 5 Gew.-% mindestens eines Vernetzungsmonomers,
g) bis zu 10 Gew.-% mindestens eines Kettenübertragungsmittels und
h) bis zu 2 Gew.-% mindestens eines polymeren Stabilisators.

25. Verfahren nach einem der vorhergehenden Ansprüche, **dadurch gekennzeichnet, dass** die Monomerenmischung Folgendes umfasst:
- ein Di(C₁-C₄)alkylamino(C₁-C₆)alkylmethacrylat,
- einen oder mehrere einfache C₁-C₃₀-(Meth)acryl-säureester,
- ein Polyethylenglykol(20-25)-C₁₀-C₃₀-alkylether-methacrylat,
- einen Polyethylenglykol/polypropylenglykol-30/5-allylether,
- ein Hydroxy(C₂-C₆)alkylmethacrylat,
- ein Ethylenglykoldimethacrylat.

26. Verfahren nach einem der vorhergehenden Ansprüche, **dadurch gekennzeichnet, dass** das bzw. die bei dem erfindungsgemäßen Verfahren verwendeten kationischen verdickenden Polymere 0,01 bis 30 Gew.-%, vorzugsweise 0,1 bis 10 Gew.-% und noch besser 0,5 bis 3 Gew.-%, bezogen auf das Gewicht der oxidierenden Zusammensetzung, ausmacht bzw. ausmachen.

27. Verfahren nach einem der vorhergehenden Ansprüche, **dadurch gekennzeichnet, dass** das bzw. die Oxidationsmittel aus Wasserstoffperoxid, Alkalimetallbromaten, Polythionaten und Persalzen, wie Perboraten, Percarbonaten und Persulfaten, ausgewählt ist bzw. sind.

28. Verfahren nach Anspruch 27, **dadurch gekennzeichnet, dass** das bzw. die Oxidationsmittel im Allgemeinen 0,1 bis 50 Gew.-% und vorzugsweise 0,2 bis 20 Gew.-%, bezogen auf das Gesamtgewicht der oxidierenden Zusammensetzung, ausmacht bzw. ausmachen.

29. Verfahren nach Anspruch 27 oder 28, **dadurch gekennzeichnet, dass** die oxidierende Zusammensetzung Wasserstoffperoxid und mindestens einen Wasserstoffperoxid-Stabilisator, ausgewählt aus Alkalimetall- oder Erdalkalimetallpyrophosphaten, wie Tetranatriumpyrophosphat, Alkalimetall- oder Erdalkalimetallstannaten, Phenacetin oder Salzen von Säuren und Oxychinolin, wie Oxychinolinsulfat, enthält.

30. Verfahren nach Anspruch 29, **dadurch gekennzeichnet, dass** der bzw. die Wasserstoffperoxid-Stabilisatoren 0,0001 bis 5 Gew.-% und vorzugsweise 0,01 bis 2 Gew.-%, bezogen auf das Gesamtgewicht der oxidierenden Zusammensetzung, ausmacht bzw. ausmachen.

31. Verfahren nach einem der vorhergehenden Ansprüche, **dadurch gekennzeichnet, dass** die bei 25°C und einer Scherrate von 1 s⁻¹ gemessene dynamische Viskosität der oxidierenden Zusammensetzung im Bereich von 50 Centipoise bis 1000 Poise und vorzugsweise von 75 Centipoise bis 100 Poise liegt.

32. Verfahren nach einem der vorhergehenden Ansprüche, **dadurch gekennzeichnet, dass** der pH-Wert der oxidierenden Zusammensetzung im Bereich von 1 bis 13 und vorzugsweise von 1,5 bis 12 liegt.

33. Verfahren nach einem der vorhergehenden Ansprüche, **dadurch gekennzeichnet, dass** man die reduzierende Zusammensetzung über einen Zeitraum von 5 bis 60 Minuten und vorzugsweise 10 bis 45 Minuten einwirken lässt.

34. Verfahren nach einem der vorhergehenden Ansprüche, **dadurch gekennzeichnet, dass** es zwischen dem Schritt des Aufbringens der reduzierenden Zusammensetzung auf das Haar und dem Fixierungsschritt einen Schritt des Erhitzens des Haars mit einem Heizeisen auf eine Temperatur zwischen 60 und 220°C und vorzugsweise zwischen 120 und 200°C umfasst.

35. Verfahren nach einem der vorhergehenden Ansprüche, **dadurch gekennzeichnet, dass** man die oxidierende Zusammensetzung über einen Zeitraum von 2 bis 20 Minuten einwirken lässt.

## Claims

1. Method for permanently reshaping keratin fibres, in particular the hair, comprising:
- a step of applying a reducing composition to the keratin fibres, to reduce the disulfide bonds in the keratin, the keratin fibres being shaped before, during or after said application, then
- a step of fixing by oxidation, to reform said bonds, by applying to the keratin fibres an oxidizing composition containing at least one oxidizing agent and at least one cationic thickening polymer which is the product of the polymerization of a mixture of monomers comprising:
a) at least one vinyl monomer substituted by at least one amine group,
b) at least one hydrophobic nonionic vinyl monomer,
c) at least one associative vinyl monomer, and
d) at least one surface-active semi-hydrophobic vinyl monomer.

2. Method according to Claim 1, **characterized in that** the reducing composition comprises, in a cosmetically acceptable medium, at least one reducing agent chosen from the reducing agents of formula
H(X')_{q}(R')ᵣ
in which X' represents P, S or SO₂, q is equal to 1, r is equal to 1 or 2 and R' is a linear or branched, saturated unsaturated, (C₁-C₂₀) hydrocarbon-based radical optionally interrupted by a heteroatom, and optionally comprising substituents chosen from a hydroxy group, a halogenated group, an amine group or a carboxy group, a ((C₁-C₃₀)alkoxy)carbonyl group, an amido group, a ((C₁-C₃₀)alkyl)aminocarbonyl group, a ((C₁-C₃₀) acyl) amino group, a monoalkylamino or dialkylamino group, a monohydroxylamino or dihydroxylamino group,
or a salt thereof in combination with a base.

3. Method according to Claim 2, **characterized in that** the reducing agent(s) are chosen from thioglycolic acid, thiolactic acid, glyceryl monothioglycolate, cysteamine, N-acetylcysteamine, N-propionylcysteamine, cysteine, N-acetylcysteine, thiomalic acid, pantetheine, 2,3-dimercaptosuccinic acid, N-(mercaptoalkyl)-ω-hydroxyalkylamides, N-monoalkylmercapto-4-butyramides or N,N-dialkylmercapto-4-butyramides, aminomercaptoalkylamides, derivatives of N- (mercaptoalkyl)succinamic acids and of N-(mercaptoalkyl)succinimides, alkylaminomercaptoalkylamides, the azeotropic mixture of 2-hydroxypropyl thioglyconate and of (2-hydroxy-1-methyl)ethyl thioglycolate, mercaptoalkylaminoamides, N-mercaptoalkylalkanediamides and formamidine sulfinic acid derivatives and salts thereof.

4. Method according to Claim 2 or 3, **characterized in that** the reducing agent(s) represent from 0.05 to 30%, preferably from 1 to 20% by weight relative to the total weight of the reducing composition.

5. Method according to any one of the preceding claims, **characterized in that** said mixture of monomers comprises, relative to the total weight of the mixture of monomers:
a) from 10 to 70% by weight of the vinyl monomer(s) substituted by at least one amine group,
b) from 20 to 80% by weight of the hydrophobic nonionic vinyl monomer(s),
c) from 0.001 to 25% by weight of the associative vinyl monomer(s),
d) from 0.01 to 25% by weight of the surface-active semi-hydrophobic vinyl monomer(s),
e) from 0 to 10% by weight of at least one hydroxylated nonionic vinyl monomer,
f) from 0 to 5% by weight of at least one crosslinking monomer,
g) from 0 to 10% by weight of at least one chain transfer agent, and
h) from 0 to 2% by weight of at least one polymer stabilizer.

6. Method according to Claim 5, **characterized in that** the mixture of monomers comprises, relative to the total weight of the mixture of monomers:
a) from 20 to 60% by weight of the vinyl monomer(s) substituted by at least one amine group,
b) from 20 to 70% by weight of the hydrophobic nonionic vinyl monomer(s),
c) from 0.01 to 15% by weight of the associative vinyl monomer(s),
d) from 0.1 to 10% by weight of the surface-active semi-hydrophobic vinyl monomer(s),
e) from 0.01 to 10% by weight of the hydroxylated nonionic vinyl monomer(s),
f) from 0.001 to 5% by weight of the crosslinking monomer(s),
g) from 0.001 to 10% by weight of the chain transfer agent(s), and
h) from 0 to 2% by weight of the polymer stabilizer(s).

7. Method according to Claim 5 or 6, **characterized in that** the mixture of monomers comprises from 1 to 5% by weight, relative to the total weight of the mixture of monomers, of the hydroxylated nonionic vinyl monomer(s).

8. Method according to any one of Claims 5 to 7, **characterized in that** the mixture of monomers comprises from 0.001 to 5% by weight, relative to the total weight of the mixture of monomers, of the crosslinking monomer(s).

9. Method according to any one of Claims 5 to 8, **characterized in that** the mixture of monomers comprises from 0.1 to 5% by weight, relative to the total weight of the mixture of monomers, of the chain transfer agent(s).

10. Method according to any one of the preceding claims, **characterized in that** the vinyl monomer(s) substituted by at least one amine group are chosen from:
- mono (C₁-C₄)alkylamino(C₁-C₈) alkyl (meth) acrylates,
- di(C₁-C₄)alkylamino(C₁-C₈)alkyl (meth)acrylates, preferably di(C₁-C₄)alkylamino(C₁-C₆)alkyl (meth)acrylates,
- mono(C₁-C₄) alkylamino (C₁-C₈) alkyl (meth) acrylamides,
- di(C₁-C₄)alkylamino(C₁-C₈)alkyl(meth)acrylamides,
- heterocyclic (meth)acrylamides containing a nitrogen atom,
- heterocyclic (meth)acrylates containing a nitrogen atom,
and mixtures thereof.

11. Method according to Claim 10, **characterized in that** the vinyl monomer(s) substituted by at least one amine group are chosen from:
- 3-(N,N-dimethylamino)propyl (meth)acrylate,
- N'-[3-(N,N-dimethylamino)propyl] (meth)acrylamide,
- 2-(N,N-dimethylamino)ethyl (meth)acrylate,
- 2-(N,N-diethylamino)ethyl (meth)acrylate,
- 2-(tert-butylamino)ethyl (meth)acrylate,
- [2-(N,N-dimethylamino)propyl] (meth)acrylamide, and
- 2-(N,N-dimethylamino)neopentyl acrylate.

12. Method according to any one of the preceding claims, **characterized in that** the hydrophobic nonionic vinyl monomer(s) are chosen from compounds having one or the other of formulae (I) and (II):
(I) CH₂=C(X)Z,
(II) CH₂=CH-OC(O)R;
in which, in each of formulae (I) and (II);
X represents H or a methyl group;
Z represents the -C(O)OR¹, -C(O)NH₂, -C(O)NHR¹, -C(O)N(R¹)₂, -C₆H₄R¹, -C₆H₄OR¹, -C₆H₄Cl, -CN, -NHC(O)CH₃, -NHC(O)H, N-(2-pyrrolidonyl), N-caprolactamyl, -C(O)NHC(CH₃)₃, -C(O)NHCH₂CH₂N-ethyleneurea, -SiR₃, -C(O)O(CH₂)ₓSiR₃, -C(O)NH(CH₂)ₓSiR₃ or -(CH₂)ₓSiR₃ group;
x is an integer ranging from 1 to 6;
each R is, independently, a C₁-C₃₀ alkyl;
each R¹ is, independently, a C₁-C₃₀ alkyl, a hydroxylated C₂-C₃₀ alkyl, or a halogenated C₁-C₃₀ alkyl.

13. Method according to Claim 12, **characterized in that** the hydrophobic nonionic vinyl monomer(s) are chosen from the C₁-C₃₀ alkyl esters of acrylic acid, the C₁-C₃₀ alkyl esters of methacrylic acid, and mixtures thereof.

14. Method according to any one of the preceding claims, **characterized in that** the associative vinyl monomer(s) are chosen from the compounds of formula (III): 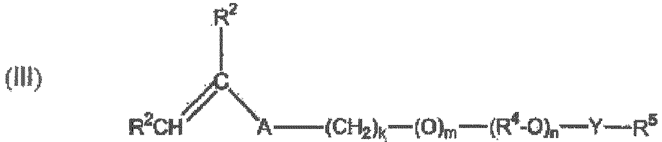 in which
each R² is, independently, H, a methyl group, a -C(O)OH group or a -C(O)OR³ group;
R³ is a C₁-C₃₀ alkyl;
A is a -CH₂C(O)O -C(O)O-, -O-, CH₂O, -NHC(O)NH-, -C(O)NH-, -Ar-(CE₂)_{z}-NHC(O)O-, -Ar-(CE₂)_{z}-NHC(O)NH- or -CH₂CH₂-NHC(O)- group;
Ar is a divalent aryl group;
E is H or a methyl group;
z is equal to 0 or 1;
k is an integer ranging from 0 to 30;
m is equal to 0 or 1, with the proviso that when k=0, m=0, and when k ranges from 1 to 30, m is equal to 1; (R⁴-O)ₙ is a polyoxyalkylene, which is a homopolymer, a random copolymer, or a block copolymer with C₂-C₄ oxyalkylene units,
R⁴ is C₂H₄, C₃H₆, C₄H₈, or mixtures thereof,
n is an integer ranging from 5 to 250,
Y is -R⁴O-, -R⁴NH-, -C(O)-, -C(O)NH-, R⁴NHC(O)NH- or -C(O)NHC(O)-;
R⁵ is a substituted or unsubstituted alkyl chosen from linear C₈-C₄₀ alkyls, branched C₈-C₄₀ alkyls, carbocyclic C₈-C₄₀ alkyls, phenyls substituted by a C₂-C₄₀ alkyl group, C₂-C₄₀ alkyls substituted by an aryl group, complex C₈-C₈₀ esters,
the R⁵ alkyl group optionally comprising one or more substituents chosen from hydroxyl, alkoxyl and halogen groups.

15. Method according to Claim 14, **characterized in that** the associative vinyl monomer(s) are chosen from polyethoxylated cetyl methacrylates, polyethoxylated cetearyl methacrylates, polyethoxylated stearyl (meth)acrylates, polyethoxylated arachidyl (meth)acrylates, polyethoxylated behenyl (meth)acrylates, polyethoxylated lauryl (meth)acrylates, polyethoxylated cerotyl (meth)acrylates, polyethoxylated montanyl (meth)acrylates, polyethoxylated melissyl (meth)acrylates, polyethoxylated lacceryl (meth)acrylates, polyethoxylated tristyrylphenol (meth)acrylates, polyethoxylated (meth)acrylates of hydrogenated castor oil, polyethoxylated (meth)acrylates of canola, polyethoxylated (meth)acrylates of cholesterol and mixtures thereof.

16. Method according to any one of the preceding claims, **characterized in that** the surface-active semi-hydrophobic vinyl monomer(s) comprise an unsaturated polymerizable end group and a polyoxyalkylene group covalently bonded to the end group.

17. Method according to Claim 16, **characterized in that** the polyoxyalkylene group is a homopolymer, a random copolymer, or a block copolymer comprising from 5 to 250 C₂-C₄ oxyalkylene units.

18. Method according to Claim 16 or 17, **characterized in that** the surface-active semi-hydrophobic vinyl monomer(s) are chosen from the compounds of formulae (IV) or (V) : 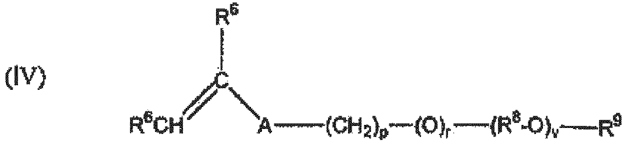
(V) D-A-(CH₂)ₚ-(O)ᵣ-(R⁸-O)ᵥ-R⁹
in which, in each formula (IV) or (V),
each R⁶ represents, independently, H, a C₁-C₃₀ alkyl, -C(O)OH or C(O)OR⁷;
R⁷ is a C₁-C₃₀ alkyl;
A is a -CH₂C(O)O-, -C(O)O-, -O-, -CH₂O, -NHC(O)NH-, -C(O)NH-, -Ar-(CE₂)_{z}-NHC(O)O-, -Ar-(CE₂)_{z}-NHC(O)NH- or -CH₂CH₂-NHC(O)- group;
Ar is a divalent aryl group;
E is H or a methyl group;
z is equal to 0 or 1;
p is an integer ranging from 0 to 30;
r is equal to 0 or 1, with the proviso that when p is equal to 0, r is equal to 0, and when p ranges from 1 to 30, r is equal to 1;
(R⁸-O)ᵥ, is a polyoxyalkylene, which is a homopolymer, a random copolymer, or a block copolymer with C₂-C₄ oxyalkylene units, in which R⁸ is C₂H₄, C₃H₆, C₄H₈, or mixtures thereof, and v is an integer ranging from 5 to 250;
R⁹ is H or a C₁-C₄ alkyl;
D is an unsaturated C₈-C₃₀ alkyl or an unsaturated C₈-C₃₀ alkyl substituted by a carboxyl group.

19. Method according to any one of Claims 16 to 18, **characterized in that** the mixture of monomers comprises a surface-active semi-hydrophobic vinyl monomer having one of the following formulae:
**CH₂=CH-O(CH₂)ₐO(C₃H₆O)_{b(}C₂H₄O)_{c}H**
or
**CH₂=CHCH₂O(C₃H₆O)_{d}(C₂H₄0)ₑH;**
in which
a is equal to 2, 3 or 4;
b is an integer ranging from 1 to 10;
c is an integer ranging from 5 to 50;
d is an integer ranging from 1 to 10; and
e is an integer ranging from 5 to 50.

20. Method according to any one of Claims 5 to 19, **characterized in that** the hydroxylated nonionic vinyl monomer(s) are chosen from the hydroxylated C₁-C₆ alkyl acrylates, preferably the hydroxylated C₁-C₄ alkyl acrylates, the hydroxylated C₁-C₆ alkyl methacrylates, preferably the hydroxylated C₁-C₄ alkyl methacrylates, the hydroxylated C₁-C₄ alkylacrylamides, the hydroxylated C₁-C₉ alkylmethacrylamides and mixtures thereof.

21. Method according to Claim 20, **characterized in that** the hydroxylated nonionic vinyl monomer is 2-hydroxyethyl methacrylate.

22. Method according to any one of Claims 5 to 21, **characterized in that** the crosslinking monomer is a polyol acrylate ester with at least two acrylate ester groups, a polyol methacrylate ester with at least two methacrylate ester groups, or a combination thereof,

23. Method according to any one of Claims 5 to 22, **characterized in that** the chain transfer agent is chosen from thiol-based compounds, disulfide compounds, phosphites, hypophosphites, haloalkyl compounds and combinations thereof.

24. Method according to any one of Claims 5 to 23, **characterized in that** said mixture of monomers comprises, relative to the total weight of the mixture of monomers:
a) from 20 to 50% by weight of at least one vinyl monomer substituted by at least one amine group chosen from:
- 3-(N,N-dimethylamino)propyl (meth)acrylate,
- N'-[3-(N,N-dimethylamino)propyl] (meth)acrylamide,
- 2-(N,N-dimethylamino)ethyl (meth)acrylate,
- 2-(N,N-diethylamino)ethyl (meth)acrylate,
- 2-(tert-butylamino)ethyl (meth)acrylate,
- [2-(N,N-dimethylamino)propyl] (meth)acrylamide, and
- 2-(N,N-dimethylamino)neopentyl acrylate,
b) from 50 to 65% by weight of at least one hydrophobic nonionic vinyl monomer chosen from the C₁-C₃₀ alkyl esters of acrylic acid, the C₁-C₃₀ alkyl esters of methacrylic acid, and mixtures thereof,
c) from 0.1 to 10% by weight of at least one associative vinyl monomer chosen from polyethoxylated cetyl methacrylates, polyethoxylated cetearyl methacrylates, polyethoxylated stearyl (meth)acrylates, polyethoxylated arachidyl (meth)acrylates, polyethoxylated behenyl (meth)acrylates, polyethoxylated lauryl (meth)acrylates, polyethoxylated cerotyl (meth)acrylates, polyethoxylated montanyl (meth)acrylates, polyethoxylated melissyl (meth)acrylates, polyethoxylated lacceryl (meth)acrylates, polyethoxylated tristyrylphenol (meth)acrylates, polyethoxylated (meth)acrylates of hydrogenated castor oil, polyethoxylated (meth)acrylates of canola, polyethoxylated (meth)acrylates of cholesterol and mixtures thereof,
d) from 0.1 to 10% by weight of at least one surface-active semi-hydrophobic vinyl monomer having one of the following formulae:
**CH₂=CH-O(CH₂)ₐO(C₃H₆O)_{b}(C₂H₄O)_{c}H**
or
**CH₂=CHCH₂O(C₃H₆O)_{d}(C₂H₄O)ₑH;**
in which
a is equal to 2, 3 or 4;
b is an integer ranging from 1 to 10;
c is an integer ranging from 5 to 50;
d is an integer ranging from 1 to 10; and
e is an integer ranging from 5 to 50,
e) up to 10% by weight of at least one hydroxylated nonionic vinyl monomer,
f) up to 5% by weight of at least one crosslinking monomer,
g) up to 10% by weight of at least one chain transfer agent, and
h) up to 2% by weight of at least one polymer stabilizer.

25. Method according to any one of the preceding claims, **characterized in that** said mixture of monomers comprises:
- a di(C₁-C₄)alkylamino(C₁-C₆)alkyl methacrylate,
- one or more simple C₁-C₃₀ (meth)acrylic acid esters,
- a C₁₀-C₃₀ alkyl ether methacrylate polyethylene glycol (20-25),
- a 30/5 polyethylene glycol/polypropylene glycol allyl ether,
- a hydroxy(C₂-C₆)alkyl methacrylate,
- an ethylene glycol dimethacrylate.

26. Method according to any one of the preceding claims, **characterized in that** the cationic thickening polymer(s) used in the method according to the invention represent from 0.01 to 30% by weight, preferably from 0.1 to 10% by weight, and better still from 0.5 to 3% by weight relative to the total weight of the oxidizing composition.

27. Method according to any one of the preceding claims, **characterized in that** the oxidizing agent(s) are chosen from aqueous hydrogen peroxide solution, alkali metal bromates, polythionates, persalts, such as perborates, percarbonates and persulfates.

28. Method according to Claim 27, **characterized in that** the oxidizing agent(s) generally represent from 0.1 to 50%, preferably from 0.2 to 20% by weight relative to the total weight of the oxidizing composition.

29. Method according to Claim 27 or 28, **characterized in that** the oxidizing composition contains aqueous hydrogen peroxide solution and at least one aqueous hydrogen peroxide solution stabilizer, chosen from the alkali metal or alkaline-earth metal pyrophosphates, such as tetrasodium pyrophosphate, the alkali metal or alkaline-earth metal stannates, phenacetin or oxyquinoline acid salts, such as oxyquinoline sulfate.

30. Method according to Claim 29, **characterized in that** the aqueous hydrogen peroxide solution stabilizer(s) represent from 0.0001 to 5% by weight, preferably from 0.01 to 2% by weight, relative to the total weight of the oxidizing composition.

31. Method according to any one of the preceding claims, **characterized in that** the dynamic viscosity of the oxidizing composition, measured at 25°C and a shear rate of 1 s⁻¹, ranges from 50 centipoise to 1000 poise, preferably from 75 centipoise to 100 poise.

32. Method according to any one of the preceding claims, **characterized in that** the pH of the oxidizing composition ranges from 1 to 13, preferably from 1.5 to 12.

33. Method according to any one of the receding claims, **characterized in that** the reducing composition is left to act for 5 to 60 minutes, preferably for 10 to 45 minutes.

34. Method according to any one of the preceding claims, **characterized in that** it comprises, between the step of applying the reducing composition to the hair and the step of fixing, a step of heating the hair with a heating iron to a temperature of between 60 and 220°C, preferably between 120 and 200°C.

35. Method according to any one of the preceding claims, **characterized in that** the oxidizing composition is left to act for 2 to 20 minutes.
